Europäisches Patentamt

(19) European Patent Office

Office européen des brevets

(11) Publication number: 0 279 994
A2

# (12) EUROPEAN PATENT APPLICATION

(21) Application number: 87311524.0

(22) Date of filing: 30.12.87

(51) Int. Cl.⁴: G01N 33/68 , //G01N33/566, G01N33/569,A61K39/00

The title of the invention has been amended (Guidelines for Examination in the EPO, A-III, 7.3).

(30) Priority: 31.12.86 US 948255
21.01.87 US 5885

(43) Date of publication of application:
31.08.88 Bulletin 88/35

(84) Designated Contracting States:
AT BE CH DE FR GB IT LI LU NL SE

(71) Applicant: THE UNITED STATES OF AMERICA
represented by The Secretary The United
States Department of Commerce
5285 Port Royal Road
Springfield Virginia 22161(US)

(72) Inventor: Berzofsky, Jay A.
9321 Corsica Drive Bethesda
Maryland 20814(US)
Inventor: DeLisi, Charles
7805 Radnor Road Bethesda
Maryland 20814(US)
Inventor: Margalit, Hanah
252 Congressional Lane Apt. 102 Rockville
Maryland 20852(US)
Inventor: Cornette, James L.
2814 Torrey Pines Circle Ames
Iowa 50010(US)
Inventor: Cease, Kemp B.
2006 Baltimore Road Apt. D43 Rockville
Maryland 20851(US)
Inventor: Spouge, John L.
800 Leverton Road Rockville
Maryland 20852(US)

(74) Representative: Jump, Timothy John Simon et
al
F.J. Cleveland and Company 40-43 Chancery
Lane
London WC2A 1JQ(GB)

(54) Method to predict antigenic sites recognized by T-lymphocytes.

(57) This invention relates to a method of predicting segments of protein sequences that are likely to be recognized by T lymphocytes and therefore to stimulate cellular immunity. The method involves determining the potential immunogenicity of certain protein sequences by using T-cell site predictors. These selected protein segments are evaluated and ranked according to the probability of the existence of a T-lymphocyte antigenic site. Peptides are thus selected as potential vaccine candidates in the treatment of infections for which T-cell mediated immunity is an important defense. Even when an antibody immunity is the critical defense, helper T cells are necessary for a memory antibody response.

# METHOD TO PREDICT ANTIGENIC SITES RECOGNIZED BY T LYMPHOCYTES SUCH AS FOR DESIGN OF VACCINES

The identification and analysis of antigenic sites on a protein and ultimately the ability to predict their location is central to a wide range of problems in fundamental and applied immunology. The molecular basis of antigen processing and recognition is an example of the former; vaccine development is an example of the latter. The emergence of hybridoma technology and the consequent availability of monoclonal antibodies have greatly facilitated the search for sites recognized by antibodies, and the antigenic architecture of a number of proteins has now been mapped in considerable detail. Careful examination of the data thus generated indicates that antigenic sites for antibodies are generally located on the protein surface in regions of relatively high segmental flexibility and hydrophilicity. The majority of the exposed surface may be antigenic for antibodies.

In contrast to the information and emerging concepts on antigenicity for antibodies, data on antigenic sites recognized by T-cells are scarce, and potentially predictive concepts are essentially nonexistent. The latter deficiency is to some extent related to the former, and both are linked to the relative complexity of the T-cell response. Unlike B-cell immunoglobulin, which can recognize native, solubilized antigen, recognition of antigen by T-cell receptors requires that it be proteolytically processed or otherwise unfolded by accessory cells, such as macrophages, B cells, or dendritic cells, and that the antigenic segments thus produced be presented to T-cells on the surface of an accessory cell in association with a major transplantation antigen, such as the murine Ia or the human HLA-D region antigens. This complexity, coupled with the fact that the T-cell receptor has only recently become amenable to structural analysis, has also made determination of equilibrium constants for binding of free antigen to T-cell receptors difficult. Thus quantitative studies of recognition by T-cell receptors, even at a phenomenological level, have progressed much more slowly than those for antibodies.

The specificity of the interaction between T-cell receptors and antigen suggests the presence of a regular order that would serve to distinguish one segment from another. Moreover, the fact that T-cell receptors do not recognize soluble antigen, but only antigen in association with a presenting cell, suggests that such order might not be present in solution but might be induced and stabilized by interactions with hydrophobic surface structures of the presenting cell. As part of the present invention, it was found that one face of a regularly ordered secondary structure consists of relatively hydrophobic residues. Polar residues on the opposite face permit the type of bonding that confers specifically on the interaction with the T-cell receptor. Also, helper T-cell immunodominant sites tend to be peptides with strong conformational propensities that stabilize under hydrophobic interaction with some structure on the antigen-presenting cell, possibly a Class II Major Histocompatibility Complex protein. Since small peptides do not commonly take stable conformations, the present invention shows that immunodominant sites are often those peptides most able to present the T-cells with a consistent conformational picture.

The strong correspondence between immunodominant T cell epitopes and segments which can form amphipathic helices suggests that this intrinsic property of the sequence may be one of the major factors determining which sites are immunodominant, in addition to any specific interaction with MHC molecules. Thus, our results suggest that having both a hydrophilic region perhaps to interact with the T cell receptor, and a hydrophobic region, perhaps to interact with the presenting cell, may be important for peptides to be optimally presented to T cells. Amphipathic structures have a natural affinity for lipid membranes. Thus, such peptides may be selected by their ability to concentrate on the membrane of the presenting cell. A high concentration may be necessary to achieve a sufficient number of peptide-MHC complexes to stimulate the T cell despite the relatively low affinity of Class II MHC for peptides. Alternatively, such amphipathic structures may preferentially interact with a hydrophobic groove or crevice in the Class II MHC molecule itself and hydrophobic interactions may account for the low degree of speficity of MHC for peptides. Although there are no strongly hydrophobic segments of sequence outside the trans-membrane region of these Class II MHC molecules, there are hydrophobic residues in the hypervariable region which could cluster in the three-dimensional structure. In either case, those sites which are not amphipathic must have some other way of binding. For instance, an extreme example is poly-L-lysine, which is not amphipathic but is so basic that it binds tightly to negatively charged groups on the cell surface. However, our results suggest that the majority of immunodominant sites known so far use amphipathic interactions instead.

With the above as background, the present invention includes statistical generalities about antigenic conformations (significances or probabilities, or, mathematically, p values) that: (1) most helper T-cell antigenic sites are amphipathic alpha-helices; (2) alphahelical amphipathicity and propensity to an alpha-helical conformation contribute independently to T-cell antigenicity; (3) there is evidence that some T-cell antigenic sites take beta formations instead of alphahelices; (4) T-cell antigenic sites avoid random coiled formations; (5) T-cell antigenic sites are usually not segmentally amphipathic; and (6) T-cell antigenic sites frequently have lysine residues near the carboxyl terminal.

## SUMMARY AND GENERAL DESCRIPTION OF THE INVENTION

The present invention is critical to the manufacture of peptide vaccines capable of eliciting T-cell immunity. One aspect of the present invention is the discovery of certain traits which seem to be common to most T-cell stimulating protein segments, such peptide vaccines should optimally utilize those protein segments which (a) have a propensity to form amphipathic alpha-helices; (b) do not have regions with a propensity to coil formations, and (c) have a lysine at their COOH-terminus. The last two observations are of particular use in manufacturing peptide vaccines: they indicate where the synthetic peptides should be terminated.

Lysines are unusually frequent at the COOH-terminal of T-cell antigenic sites, even after accounting for tryptic digests. These lysines can stabilize alpha-helical peptides by a favorable interaction with alpha-helical dipoles. This interaction, which occurs with other charged residues and not just lysine, is probably stronger in peptides than in native proteins because of the terminal back-bone charges in free peptides. This stabilization may explain why deletion or replacement of COOH-terminal lysines often destroys antigenic activity, an experimental fact never before noted as a general observation.

The ability to predict T-cell antigenic peptides has important implications for the development of artificial vaccines. The present invention is a new technique for predicting and identifying peptide segments (of protein antigens) that are likely to elicit T-cell immunity. It was developed using, in part, Monte Carlo computer experiments which are applicable to many problems involving protein or DNA.

The technique is used to evaluate the contribution of various peptide properties to helper T-cell antigenicity. The properties investigated include amphipathicities (alpha and beta), conformational propensities (alpha, beta, turn and coil) and the correlates of alpha-helices, such as the absence of helix-breakers and the positioning of the residues which stabilize alpha-helical dipoles. We also investigate segmental amphipathicity (a peptide has this property when it contains at least two disjoint subpeptides, one hydrophobic, one hydrophilic). Statistical correlations and stratifications assessed independent contributions to T-cell antigenicity.

## DESCRIPTION OF THE FIGURE

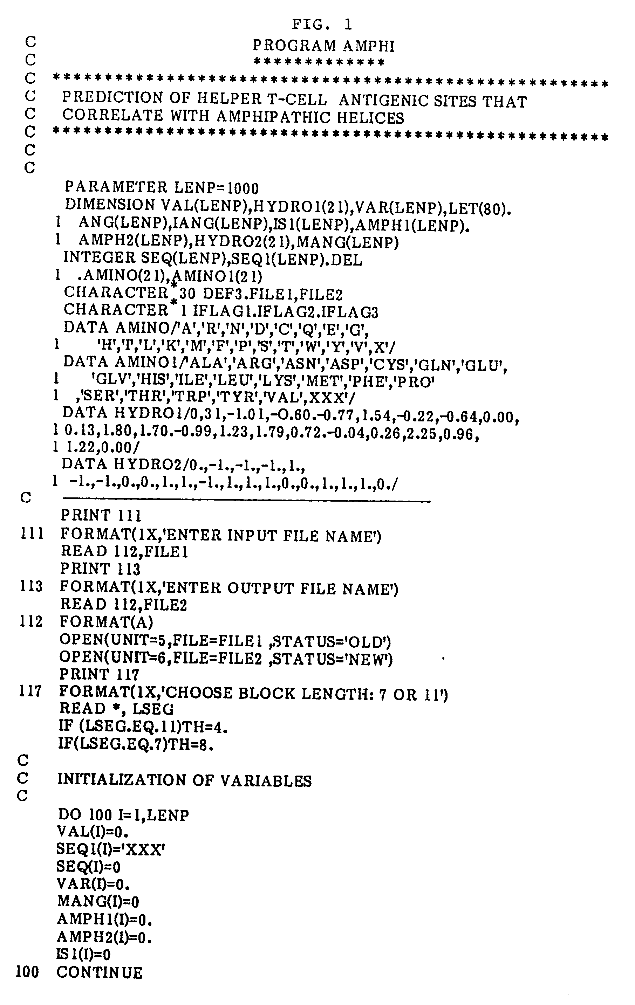

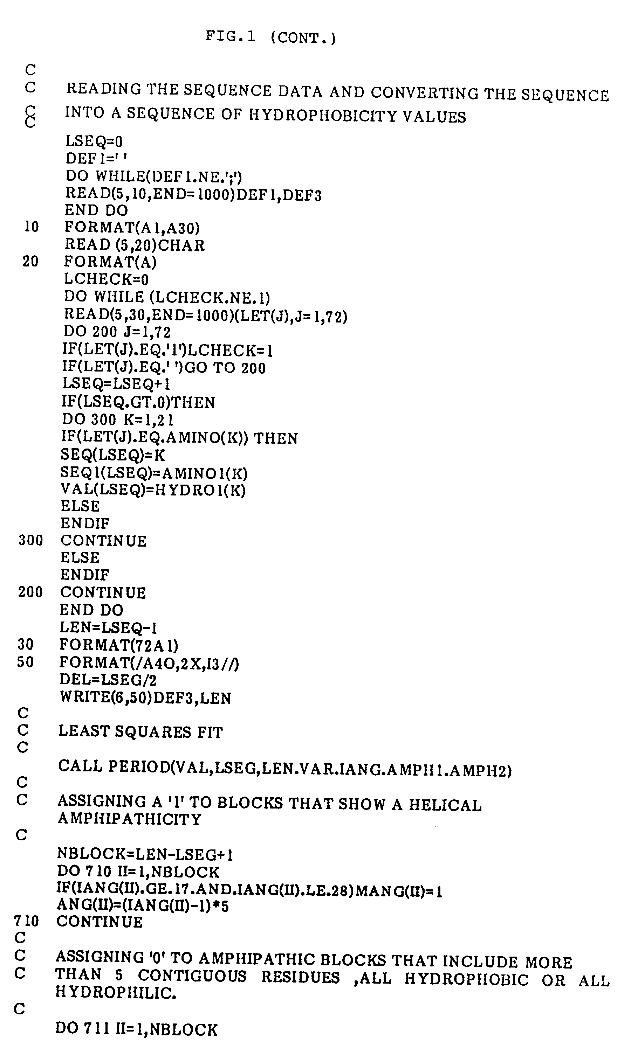

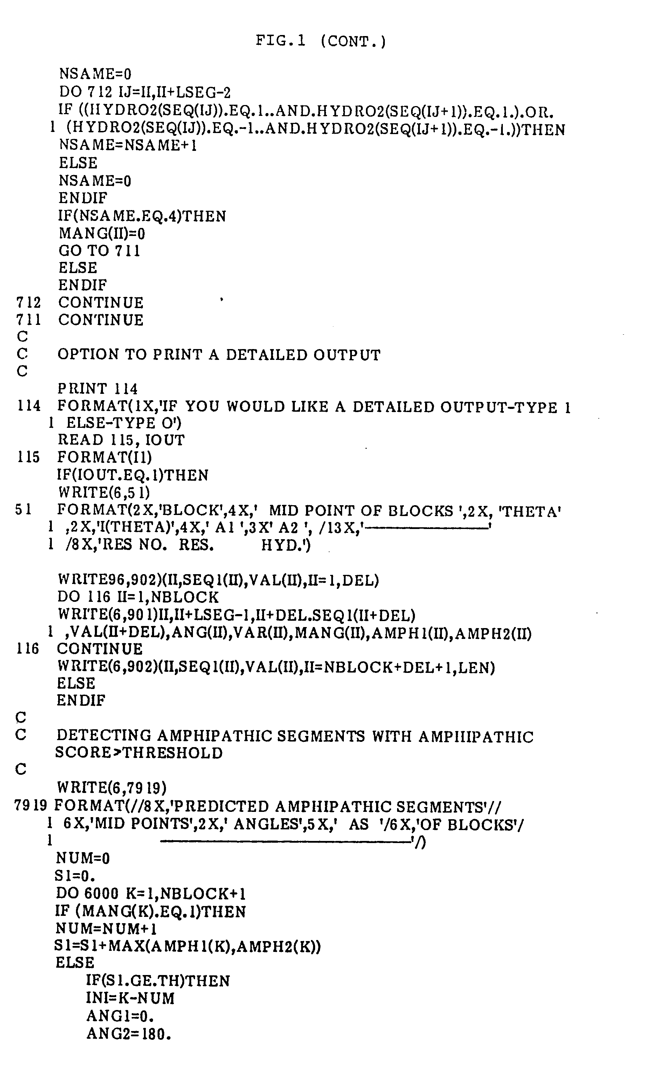

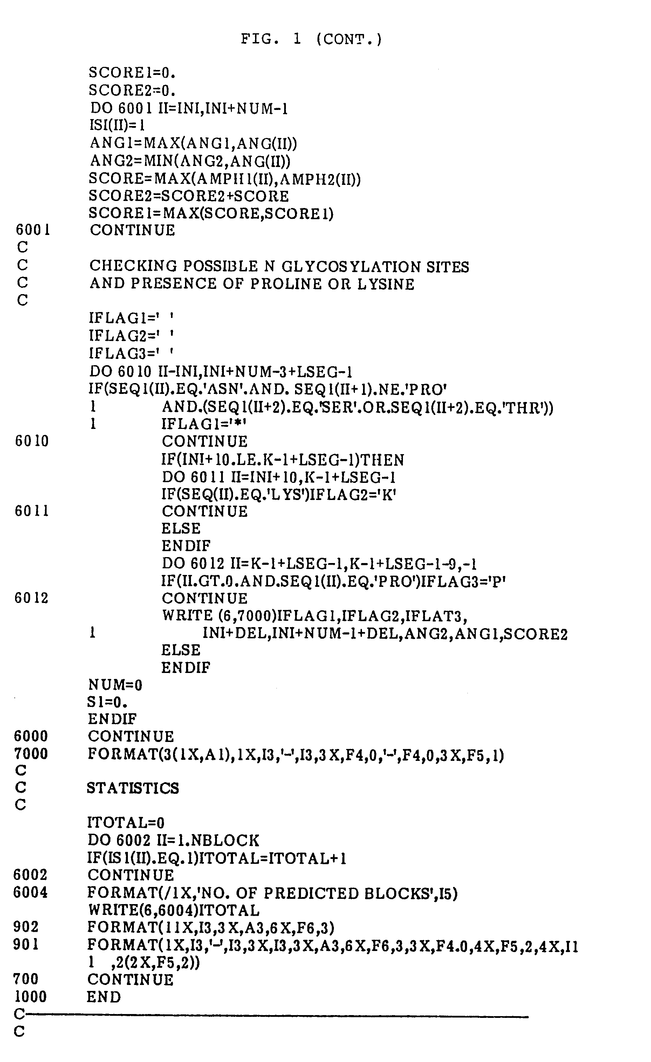

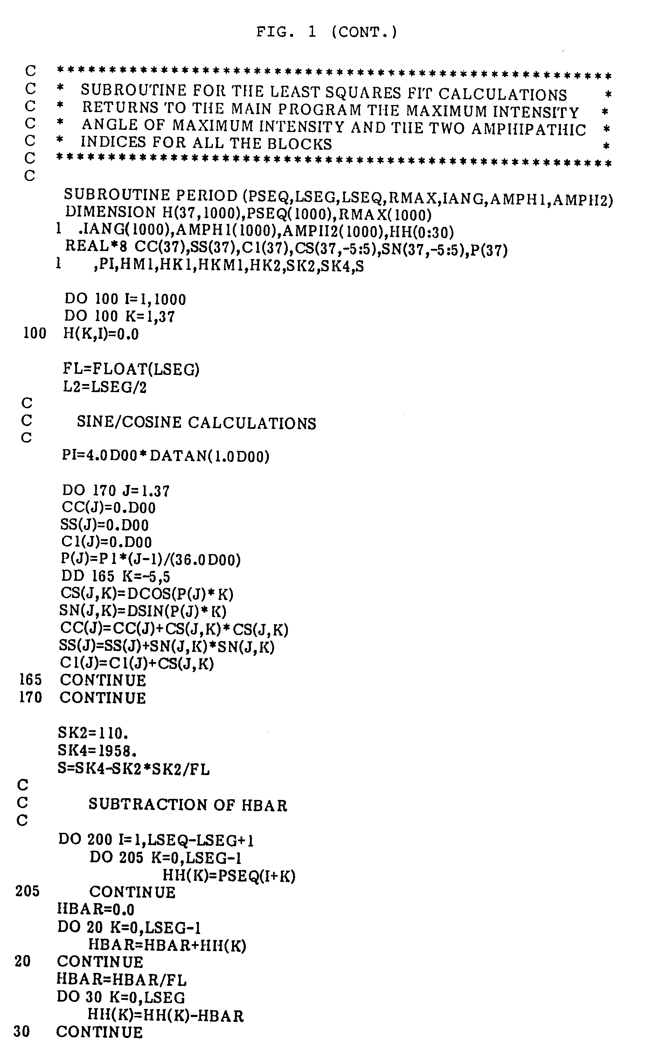

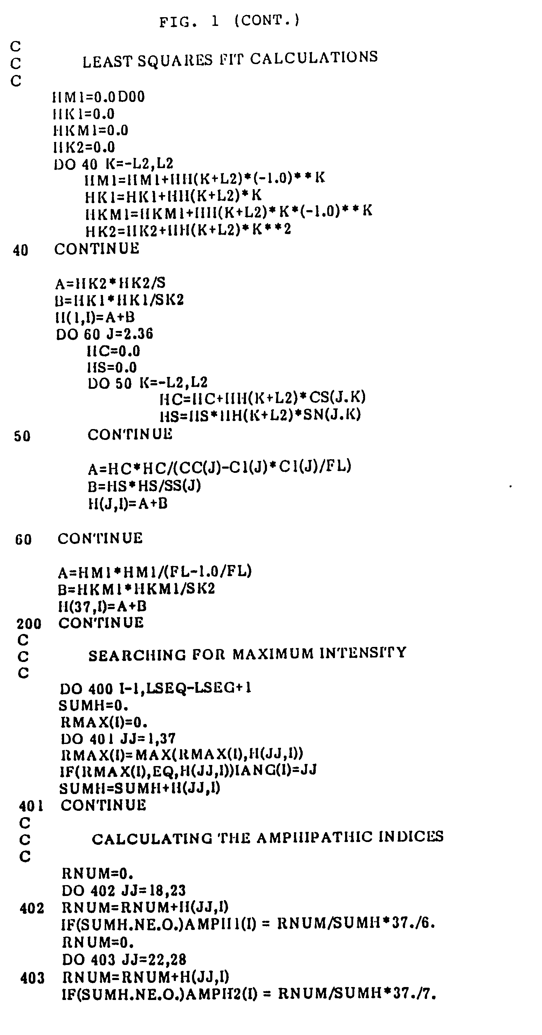

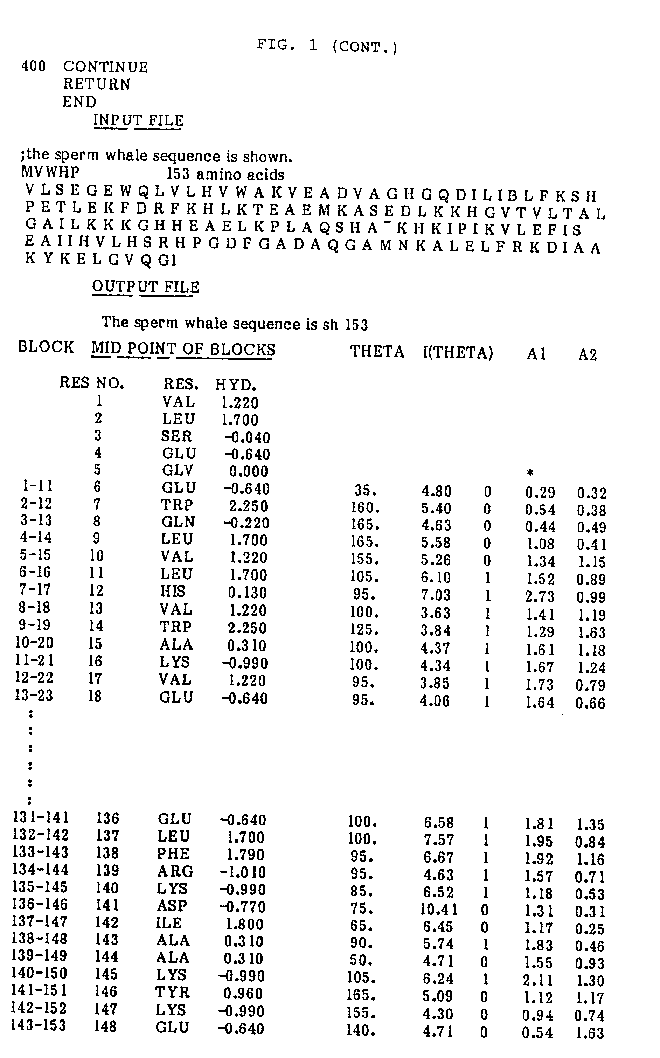

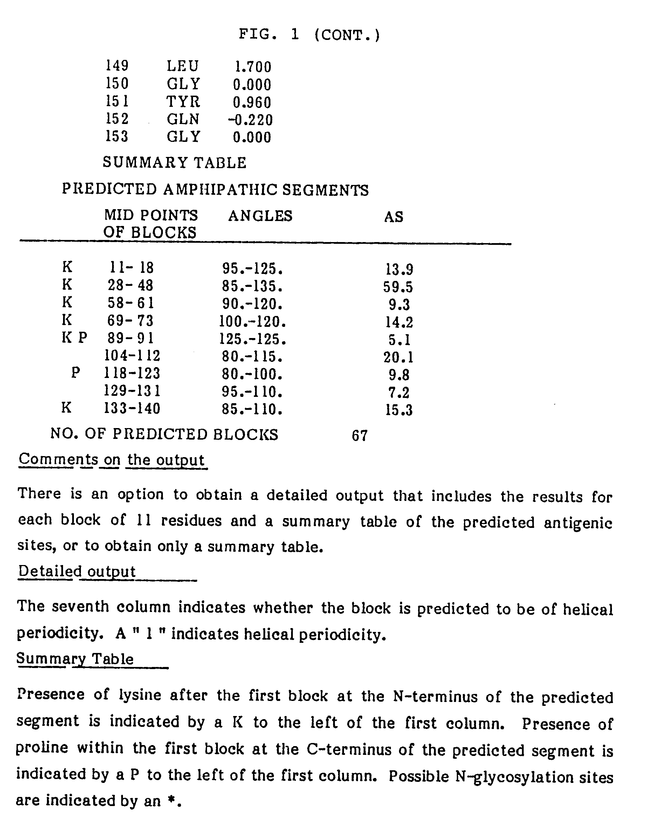

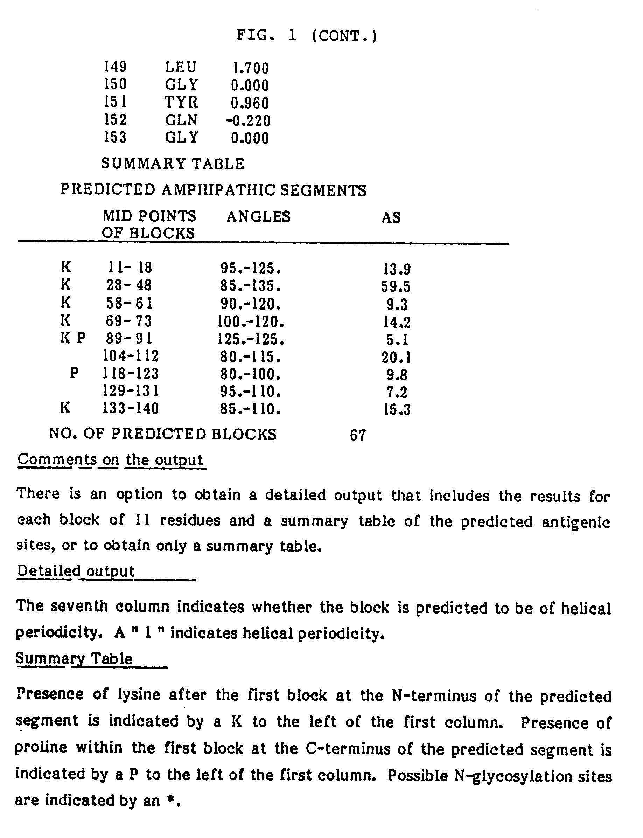

Figure 1 shows a computer program embodying part of the present invention.

## DETAILED DESCRIPTION OF PREFERRED EMBODIMENTS OF THE INVENTION

Prediction of T-cell antigenic peptides has important implications for the development of artificial vaccines. Such vaccines are particularly useful in diseases like leprosy, caused by organisms which are hard to culture and for which the cellular arm of the immune system is the principal defense. Even when antibody production is the primary goal of vaccination, a secondary or anamnestic response requires the induction of helper T-cell immunity. Predeiction of peptides for use as vaccines requires discovery and confirmation of properties correlating the T-cell antigenicity. One of the purposes of this invention is to use such properties in a process capable of reliably predicting T-cell stimulation by a protein segment.

The experimental peptides containing the immunodominant sites are defined herein as antigenic sites. "Antigenicity" in this invention always refers to T-cell antigencity.

In vivo, an antigenic protein probably passes through three main steps before raising a helper T-cell response: (a) "processing": an antigen-presenting cell (APC), usually a macrophage, dendritic or B cell, ingests the protein and then digests it into smaller peptides; (b) "presentation": these peptides are then presented to T-cells, probably in conjunction with a Class II Major Histocompatibility Complex Protein on the APC surface; and (c) "recognition": a helper T-cell receptor then recognizes some combination of peptide and Class II Protein, and initiates a T-cell response.

Two antigenic properties are thought to contribute to this process, amphipathicity and alpha-helicity, based on the findings of this invention.

A structure is amphipathic when it has both a hydrophobic portion and hydrophilic portion. A peptide is segmentally amphipathic when the peptide contains at least two disjoint subpeptides, one hydrophobic, the other hydrophilic. A peptide is alpha-amphipathic if, when the peptide is put into an alpha-helical conformation, one side of the alpha-helix is hydrophobic, the other side hydrophilic. A peptide is helically amphipathic if, when put into an alpha or $3_{10}$-helix, or similar helical structure, one side of the helix is hydrophobic and the other side is hydrophilic. Both segmental amphipathicity and helical amphipathicity are believed to contribute to T-cell antigenicity, though opinions about their relative importance differ.

Much is known about the alpha-helical conformation. Certain amino acids are helix-makers, e.g., glutamate; others are helix-breakers, e.g., proline, glycine, and serine. Because of the orientation of the peptide bonds in their backbones, alpha-helices have an intrinsic dipole equivalent to a charge of about $+\frac{1}{2}$ e at the $NH_2$-terminus and $-\frac{1}{2}$ e at the COOH-terminus (e = elementary charge). The dipole exists even when the alpha-helix is part of a longer peptide. Negatively charged residues (Asp/Glu) at the $NH_2$-terminus interact favorably with the dipole, as do positively charged residues (Arg/His/Lys) at the COOH-terminus. These interactions can help to stabilize an alpha-helix, and in fact, many alpha-helices in native protein have these residues in the appropriate position. Alpha-helicity, if present, can have many implications for the composition of antigenic peptides.

The extended (i.e., beta) peptide conformation is common in native proteins and can also be amphipathic. Unlike alpha-amphipathicity, beta-amphipathicity is not yet implicated in T-cell antigenicity. Beta-propensity is used to connote a tendency to beta conformation. Similarly, peptides with a tendency to alpha-helicity have alpha-propensity. Turn propensity is the tendency to form a beta turn; coil propensity is the propensity to form a random coil.

If an antigenic site was produced by a tryptic digest, then the corresponding random site should end in arginine or lysine; likewise, it is was due to a cyanogen bromide reaction, then the corresponding random site should end in a methionine. We call this Cleavage Restriction.

Tryptic digests (which force the terminal residue of an antigenic site to be either arginine or lysine) systematically bias the COOH-terminal residue of an antigenic site. Lysines at the COOH-terminus of antigenic sites turn out to be important. Cleavage Restriction controls the bias that tryptic digests and cyanogen bromide reactions introduce into the COOH-terminal residues.

Confirmation of the correlation of amphipathicities, propensities and other properties with immunodominance requires a statistical test. Classical statistical methods are inappropriate for protein analysis because they require analytic description of the parent distribution. The present invention provides a novel and appropriate statistical test for significance in protein (or DNA) data bases, made practicable by Monte Carlo computer experiments. This test confirms the correlation between a property and peptide antigenicity.

An algorithm is defined as a unique combination of the block length, the hydrophobicity scale, and the criterion for detecting stable amphipathic segments.

The present invention is a method of predicting which segments of a protein (along its entire sequence, if desired) are antigenic. In other words, the present invention is a method of determining which sites of an entire protein sequence are recognized by T-cells (activate or stimulate T-cells). Application of this method is limited only by knowledge of the amino acid sequences of a protein, i.e., can be applied to any protein in the protein data base of the National Biomedical Research Foundation or any protein whose sequence is subsequently published. Moreover, the analysis can be done without isolating the protein by using the amino acid sequence translated from a DNA gene sequence. The background experiments which made this process possible comprise, in their entirety, an examination of a number of properties to determine if a particular property of properties is implicated in T-cell stimulation. Table 2 lists the properties examined.

The following properties were determined to be fundamentally important (with a high degree of significance) in determining the potential immunogenicity of certain protein sequences:

a. the helical amphipathicity of segments along the entire sequence of a protein (see Example 1);

b. the conformational propensity of segments along the entire sequence of the protein (see Example 2);

c. the presence or absence of helix-breakers in segments along the entire sequence of the protein (see Example 3); and

d. the presence and location in the protein sequence of amino acid residues which favor T-cell recognition (see Example 4).

These properties, as well as others noted in Table 1, were used to develop an optimized algorithm for detecting T-cell antigenic sites (based on the amphipathic helix model) in a protein with known sequences. The optimum algorithm identifies 18 of 23 known sites (75% sensitivity), with a high degree of significance (p.0.001). The success of the algorithm also shows that stable amphipathic structures such as amphipathic helices are fundamentally important in determining immunodominance. The optimized algorithm, with the

4

computer program listed in the Figure and Example 9, enables the prediction of immunodominant T-cell sites on a protein. This prediction capability facilies the rational design of synthetic vaccines, and facilities other approaches to antigen specific T-cell recognition.

The general scheme for all the algorithms is: I) Convert the amino acid sequence into a sequence of hydrophobicity values; II) Divide the hydrophobicity sequence into overlapping blocks; e.g., a sequence L long contains $L-\ell +1$ overlapping blocks of length $\ell$ ; III) In each of the blocks, search for periodicity in hydrophobicity consistent with a regular amphipathic helical structure; IV) Distinguish between stable and unstable amphipathic helical segments; V) Search for the presence and location of amino acid residues which favor T-cell recognition. The various algorithms differ by the block length, the hydrophobicity scale, or by the definition of a stable amphipathic segment. Several ranges of angles consistent with different types of helices are also considered. We examine the power of each algorithm as a predictive tool.

Prediction of amphipathic helical segments

The basic procedure for searching for amphipathic segments along the amino acid sequence of a protein is to apply a power spectrum procedure on the appropriate sequence of hydrophobicity values. There are two possible procedures for calculating the power spectrum: the discrete Fourier transform and a least squares fit of a sinusoid (Cornette et al., manuscript submitted).

Let $h_1$, $h_2$. . .$h_L$ denote the sequence of amino acid hydrophobicities (according to a particular scale) of a protein L residues long. Divide the sequence into $L-\ell +1$ overlapping blocks of length $\ell$. The first block extends from residue 1 to residue , the second block from residue 2 to $\ell +1$, etc. Let $h_k$ be the average hydrophobicity of the $k_{th}$ block (k, k+$\ell$-1). For the discrete Fourier transform the intensity corresponding to the $k^{th}$ block is

$$I(k,\Theta) = \left\{ \left[\sum_{j=k}^{k+\ell-1} (h_j-\bar{h}_k) \sin(2\pi\Theta j/360)\right]^2 + \left[\sum_{j=k}^{k+\ell-1} (h_j-\bar{h}_k) \cos(2\pi\Theta j/360)\right]^2 \right\}^{1/2} \quad (1)$$

while, when using a least squares fit, the intensity function is calculated by

$$I(k,\Theta) = \frac{\left[\sum_{j=k-\ell_1}^{k+\ell-1} (h_{j+\ell_1}-\bar{h}) \cos j\Theta\right]^2}{\sum_{j=k-\ell_1}^{k+\ell_1} \cos^2 j\Theta - \frac{1}{\ell}\left[\sum_{j=k-_1}^{k+_1} \cos j\Theta\right]^2} + \frac{\left[\sum_{j=k-\ell_1}^{k+\ell-1} (h_{j+\ell_1}-\bar{h}) \sin j\Theta\right]^2}{\sum_{j=k-\ell_1}^{k+\ell_1} \sin^2 j\Theta} \quad (2)$$

where $\ell_1$ is equal to $(\ell-1)/2$ and the block length, $\ell$, is an odd number. Since in the present analysis we use only odd block lengths, this formula is satisfactory.

Both, (1) and (2), are computed for $\Theta = 0°$, 5°, 10°, ..., 180°. The blocks for which the maximum intensity occurs at a frequency $\Theta$ near 100° reflect a periodicity of 3.6 residues per turn (360°/$\Theta$), i.e., the structure of an amphipathic alpha helix. A block is considered to be of an alpha-helical periodicity if the maximum intensity occurs at a frequency, $\Theta$, in the range 80°-120°. Since in several cases $3_{10}$ helices (periodicity of 120°, i.e. 3 residues per turn) are shown to appear at the ends of alpha-helices, and those might overlap as well with known antigenic sites, we extended the analysis to search also for such structures. A block is considered to be of a $3_{10}$ periodicity if the intensity maximizes between 105°-135°. Because there is an overlap between the two ranges of angles for the two types of periodicities, and since a careful screening of the dominant power spectrum frequencies of all the blocks revealed that alternations between the two extremes of small angles corresponding to an alpha-helix (80°-90°) and large angles corresponding to $3_{10}$ helix (125°-135°) were very rare between two adjacent blocks, we found it legitimate to search for a maximum intensity between 80°-135°, which then corresponds to a helix of either type. Implicit in this analysis is the capability of a specific sequence of residues to form a helical structure. Then the analysis asks whether this sequence, if folded as a helix, would form an amphipathic one. It is possible that a block might show the desired periodicity but have hydrophobicity values which merely fluctuate between larger and smaller values of the same sign (i.e., all hydrophobic or all hydrophilic). Such helices do not have a hydrophobic surface and a hydrophilic surface and cannot be considered an amphipathic. Thus we do not consider a block as amphipathic when it contains five or more consecutive residues of the same sign. (For this purpose <u>only</u> we treat Alanine, Glycine, Histidine, Serine and Threonine as neutral since they are not strongly hydrophobic or hydrophilic according to most scales and their hydrophobic/hydrophilic character is somewhat ambiguous. For instance, we would not like to exclude an otherwise good amphipathic helix because it had a serine on the the hydrophobic side or an alanine on the hydrophilic side.)

After detecting the blocks with the right periodicity we have to define which of them can be considered to form stable amphipathic helical segments. Two schemes, based on two different approaches are tested.

(a) We consider an amphipathic segment a stable one if it contains at least a certain number of consecutive blocks of the right periodicity. The smallest number of required consecutive blocks tested in the analysis is three. The rationale for this constraint is that three consecutive amphipathic helical blocks ensure that the intermediate block contains residues which contribute to the helical conformation of the adja cent blocks, and therefore is considered to be more helically stable than an isolated block of the right periodicity. Algorithms requiring strings of 3, 4 or 5 consecutive blocks of correct periodicity are tested.

(b) We developed a quantitative measure for evaluating the amphipathic character of a segment, which combines the length of the segment and the magnitude of intensities around 100° or around 120° of the individual blocks into an amphipathic score. A segment which has an amphipathic score above a certain threshold is considered to be a stable one. Here, the rationale is that a longer segment of weak amphipathic blocks may be stabilized by mutual reinforcement and so compare with a shorter one of higher intensity blocks.

Evaluation of the intensity of a segment is done as follows: an alpha-helical amphipathic index (Cornette et al., manuscript submitted) and a $3_{10}$ helical amphipathic index corresponding to the individual blocks are defined. The amphipathic index expresses the magnitude of the intensity peak in the power spectrum around the appropriate periodicity (100° in the case of an alpha-helix and 120° in the case of a $3_{10}$ helix) in comparison to the intensities of the whole spectrum of angles (0°-180°).

The amphipathic index around 100° (A1) for the $k^{th}$ block is defined as:

$$A1(k) = \frac{\frac{1}{6} \sum_{\Theta=85°}^{110°} I(k,\Theta)}{\frac{1}{37} \sum_{\Theta=0°}^{180°} I(k,\Theta)}$$

and the amphipathic index around 120° (A2) is defined in a similar way:

$$A2(k) = \frac{\frac{1}{7} \sum_{\Theta=105°}^{135°} I(k,\Theta)}{\frac{1}{37} \sum_{\Theta=0°}^{180°} I(k,\Theta)}$$

The amphipathic index of a block (A) is defined as the maximum of the two amphipathic indices of the block. $A(k) = \max(A1(k), A2(k))$

The amphipathic score (AS) of a segment that contains blocks with maximum intensity between 80° and 135° is then the sum of the amphipathic indices of the blocks:

$$AS = \sum_{k=n_1}^{n_2} A(k)$$

when the amphipathic segment is from the $n_1^{th}$ block to the $n_2^{th}$ block.

## Definition of a match

When a block length of 11 is used in the analysis, an antigenic site is defined as overlapping with an amphipathic segment if at least one block of that segment (i.e. all 11 residues) is fully contained within the antigenic site. For a block length of 7, we consider a match between the antigenic site and the amphipathic segment if at least two consecutive blocks of the amphipathic segment are fully included in the antigenic site.

## Probability calculations

For each antigenic site $\sigma$, we derive a sequence of +'s and -'s which identifies predicted antigenic sites of the same length $\lambda\sigma$ as that of site $\sigma$. Let the protein containing $\sigma$ have length L. Within the protein there are $L-\lambda\sigma+1$ possible positions for placement of a site of length $\lambda\sigma$. In each possible position for the site, we put a + if the site when placed there is predicted by the algorithm to be antigenic (according to the definition of a match for the different block lengths). Otherwise, we assign the position a -.

Then $\pi\sigma$, the proportion of +'s in the antigenic +/- sequence corresponding to $\sigma$, is the probability that an antigenic site $\sigma$ will happen to fall in an amphipathic region of that protein by chance alone. This assumes that each possible antigenic position has an equal chance of being chosen. Spouge et al., J. Immunol., 138:204-212 (1987) examines the implications of this assumption at greater length.

When the block length is greater than 25, performances of the discrete Fourier transform and the least squares fit are approximately the same when prediction of amphipathic alpha-helices is tested. When the block length is less than or equal to 25, the least squares fit performs better in detecting amphipathic alpha-helices (Cornette et al, manuscript submitted). Although in the present analysis the block length tested was not greater than 11, we examined both procedures, the discrete Fourier transform and the least squares fit.

The various algorithms differ by any of the following:

1) Block length -two block lengths are tested: $l=7$ and $l=11$. The former may correspond to two turns of an alpha-helix of a $3_{10}$ helix, while a block of 11 residues covers approximately 3 turns of an alpha-helix of 4 turns of a $3_{10}$ helix.

2) Hydrophobicity scale -there are many published hydrophobicity scales, some of which are better than others in detecting amphipathic structures in amino acid sequences.Among them, it must be determined which one will best serve the purpose of predicting amphipathic helical antigenic sites. The scales can be divided into two groups: (1) those derived from the chemical behavior of each amino acid (experimental scales), and (2) those based on the locations of the different amino acids in the native protein molecules for which crystallographic data were available (statistical scales). A thorough analysis of 38 hydrophobicity scales carried out by Cornette et al. (manuscript submitted) revealed three preferred scales for detecting amphipathic alpha-helices in proteins: the statistical scale of Miyazawa and Jernigen, Macromolecules, 18:534 (1985); the statistical scale of Rose et al, Science, 229:834 (1985); and the experimental scale of Fauchere and Pliska, Eur. J. Med. Chem., 18:369 (1983).

Tables II-IV summarize the total number of predicted amphipathic helical segments among the 12 proteins, the number of predicted antigenic sites out of the 23 known T-cell sites and the probability of getting this number of matches by chance alone, for various algorithms. Comparison between the performances of the least squares fit procedure and the discrete Fourier transform shows that, for almost all the algorithms, the least squares fit procedure gives better results than the discrete Fourier transform. COmparing the performances of the several hydrophobicity scales shows that either when using the least squares fit or when using a discrete Fourier transform, for all the combinations of a criterion for a stable amphipathic segment and a block length, the highest number of correct identifications of antigenic sites with the lowest probability are achieved when the hydrophobicity values of the Fauchere-Pliska scale are used. These results for some of the algorithms are summarized in Table III. These two comparisons indicate that an efficient predictive algorithm should be one that uses a least squares fit of a sinusoid as the power spectrum procedure, with the hydrophobicity values of the Fauchere-Pliska scale.

Table IV presents the results for different combinations of block lengths and criteria for detecting stable amphipathic helices when applying a least squares fit and using the hydrophobicity values of the Fauchere-Pliska scale. Comparison between the different criteria for detecting stable amphipathic segments suggests that the scheme using the amphipathic score as a criterion should be preferred. For a block length of 11 the highest number of correct identifications with the lowest probability is achieved by the scheme that requires the amphipathic score to be greater than 4. This algorithm predicts 18 out of the 23 known antigenic sites with a chance probability of 0.0006. For block length of 7 the scheme that uses a threshold of 8 for the amphipathic score gives the best result, 17 correct identifications with a chance probability of 0.0025.

Setting the threshold to 4 may not be required for every protein that will be analyzed. From a practical point of view out interest is to predict the segments that are most capable of raising a T-cell response for a specific protein. For a protein which is rich in amphipathic sites, the segments with an amphipathic score above 8 should be considered as the best candidates, while for a protein with a low content of amphipathic segments a threshold of 4 should be used.

The algorithm is not merely predicting alpha-helices. Only about half of the crystallographically-defined alpha-helices are amphipathic (Cornette et al., manuscript submitted). In addition, not all the predicted sites correspond to known helices in the native protein. By using the optimal algorithm, 13 out of the 18 identified sites are found to have alpha-helical periodicity and 5 to have $3_{10}$ helical periodicity. Out of the 13 alpha-helical sites, 8 are known to overlap (at least partially) with known alpha-helices in the native structure. These are the three sites of sperm whale myoglobin: 69-78, 102-118, 132-145; one of the pork insulin sites: residues 5-16 of the B chain; two of the chicken lysozyme sites: 74-86 and 81-96; the beef cytochrome site: 66-80; and the lambda repressor site: 12-26. In addition, residues 93-104 of pigeon cytochrome c are probably also in an alpha-helical conformation, due to the high degree of sequence similarity to cytochromes c of other species which are known crystallographically to be alpha-helical in this region, and according to minimal energy calculations showing that the most stable configuration for this segment is alpha-helical. The structure in the native protein of the other sites with alpha-helical periodicity has not yet been studied experimentally, However, in the case of ovalbumin, additional evidence that this site may form an amphipathic alpha-helix has been presented. The predicted $3_{10}$ helices overlap with the sites 109-119 and 302-313 of influenza hemagglutinin, 38-52 of hepatitis B virus major surface antigen, 11-25 of beef cytochrome c and 32-44 of rabies virus spike glycoprotein precursor. While the native structure of the latter three is know known, it is known from crystallographic data that the influenza sites are not helical. However, although those are not helices in the native structure, the T cell may never have a chance to see the native structure. Short peptides resulting from antigen processing, corresponding to these segments, have the capability of forming an amphipathic helical structure in the appropriate environment, i.e. the anisotropic environment at the interface on the surface of the presenting cell and/or in association with an MHC molecule, which should stabilize a conformation which is amphipathic.

Monte Carlo computer experiments

Any quantifiable parameter suggested to correlate with antigenicity may be evaluated as follows: define $S_0$ as the sum of the values of this parameter for all known antigenic sites in the data base. Let S denote the sum of such values for the same number of randomly selected segments of the same length in the same proteins. The significance of the parameter, which is the probability that S exceeds $S_0$, can be done by a computer employing Monte Carlo computer experiments. The computer chooses random sites a large number of times. Each time the 'random' overall statistic S is computer and compared to $S_0$. The proportion of times that S is greater than or equal to $S_0$ is the required estimate of the statistical significance of $S_0$. The more times the computer chooses random sites, the better this estimate of significance. Each event is one binomial trial, a 1 ($S_0 < S$) or a 0 ($S \leq S_0$), and an appeal to the binomial distribution shows that 50,000 computer trials give an estimate of significance accurate to about 0.005. Accordingly, this was the number of trials used.

The significances for various statistics appear in Table I, while correlations and their significances are in Table VIII. Both of these tables were obtained under Cleavage Restriction.

The results in Tables I and VIII have important implications for the manufacture of peptide vaccines. As is noted above, peptides which are potential candidates for devlopment into vaccines should, if possible, be those protein segments (a) which have a propensity to form amphipathic alpha-helices, (b) which do not have regions with a propensity to coil conformations, and (c) which have a lysine at the COOH-terminus. The last two observations are of particular use in manufacturing peptide vaccines: they indicate where the synthetic peptides should be terminated.

EXAMPLES

EXAMPLE 1. Alpha-Amphipathicity

The first property to be examined is alpha-amphipathicity. The intensity of the discrete Fourier transform provides a site statistic. The Fourier transform picks out periodicities in a sequence of numbers: in this case, it can pick out the 100° periodicity of hydrophobicities corresponding to an amphipathic alpha-helix. (a) Divide the proteins into overlapping blocks of length $l$. The first block extends from residue 1 to residue $l$, the second block from residue 2 to residue $l$ + 1, etc.. [If the protein has length L, then the number of blocks is L-$l$ + 1 (e.g., a protein of length $l$ contains exactly 1 block).] $l$ = 11 is appropriate, since Fourier transforms with smaller $l$ do not always reflect periodicities faithfully (Cornette et al., manuscript submitted). Since two minimal antigenic sites, Sperm Whale Myoglobin 69-78 and Influenza Hemagglutinin 111-119 in Table I are of length less than 11, these sites are extended for amphipathicity statistics only to make their lengths 11. The resulting peptides retain near-maximum antigenicity. The NH$_2$-terminus rather than the COOH-terminus was extended because, as is shown later, COOH-terminal lysines correlate with antigenicity.

Let $h_k$ be the hydrophobicity of the $k^{th}$ residue in the protein and $\bar{h}_k$ be the average hydrophobicity of the $k^{th}$ block (which consists of residues k to k + -1). The intensity of the discrete Fourier transform of the residue hydrophobicities is

$$I(k,\Theta) = \left\{ \left[ \sum_{j=k}^{k+ -1} (h_j - \bar{h}_k) \sin(2\pi\Theta j/360) \right]^2 + \left[ \sum_{j=k}^{k+ -1} (h_j - \bar{h}_k) \cos(2\pi\Theta j/360) \right]^2 \right\}^{1/2} \quad (1)$$

The Fourier intensity can again be converted to site statistics in many different ways. The Maximum alpha-Intensity is an appropriate choice: (b) For each block, take the maximum of the Fourier intensities at $\underline{\Theta}$ = 80°, 85°, 90°, ..., 120°. [Unlike the counterpart statistic in (7), the Maximum alpha-Intensity does not depend on values outside the 80° to 120° range.] Because the Fourier intensity at 100° corresponds to the amphipathicity of residues in an exact alpha-helical conformation, the maximization around 100° producing the Maximum alpha-Intensity allows for deviation from exact alpha-helicity. This maximization provides a block statistic which is then Block Maximized (see Example 7) to yield a site statistic. Because Maximum alpha-Intensity is the only statistic we use to represent alpha-amphipathicity for the Monte Carlo experiments, we shall refer to it as "alpha-Amphipathicity" (A).

Several general criteria for selecting amphipathic segments were chosen (see "Prediction of amphipathic helical segments", above, and Example 9). An amphipathic segment should contain a minimal number of adjacent blocks, preferably, 3-5. Also, the amphipathic score of a predicted segment should be above a certain threshold--by setting the minimal value for the threshold to 4 (for the least squares fit procedure), the chance that an isolated block of the correct periodicity is ked as an amphipathic segment is less than 0.02%. By similar analysis for the discrete Fourier transform, the lowest threshold when using this procedure should be 3.

EXAMPLE 2. Conformational Properties

All of these were strongly represented in the antigenic sites, suggesting that many antigenic sites take an alpha conformation. Of these properties, alpha-Amphipathicity was the most significant. The correlation between alpha-amphipathicity and alpha-propensity had a significance of p = 0.136, suggesting that the two properties may make independent contributions to T-cell antigenicity.

Alpha-properties.

A consistent significance for alpha-properties emerges, suggesting that most T-cell antigenic sites take an alpha-helical conformation. Alpha-amphipathicity and alpha-propensity are both significant (p = 0.017 and p = 0.136). Hence, alpha-amphipathicity may be a significant factor in T-cell antigenicity independent of its correlation with alpha-propensity. Antigens stimulating helper T-cells may bind to the Class II protein through hydrophobic interaction; because recognition occurs at the interface between a Class II protein at the antigen-presenting cell surface and an aqueous environment, alpha-helical amphipathicity may help to stabilize antigens in alpha-helical conformation. This forms the basis of the so-called amphipathicity hypothesis.

Alpha-Helical Properties.

The alpha-helical conformation is well-investigated and as such has many different measures and implications. The number of statistics presented reflect this depth.

i. Alpha-Propensity. (a) Divide the proteins into overlapping blocks of length $l$ ($l = 9$ since this is the length of the shortest antigenic site). (b) Sum the appropriate values in Table 1 of Garnier, Osguthorpe and Robson, J. Mol. Biol., 120:97 (1978) (which we refer to as G-O-R Table 1) to calculate the directional alpha-helical information for the central ($5^{th}$) residue in the block. This generates a block statistic. In a departure from the usual procedure, Block Average to produce a site statistic. This gives the tendency of the entire site to form an alpha-helix. (Block Maximization would reflect the residue most likely to be in alpha-helical conformation; if isolated, this residue is probably not very important.) Because this statistic attempts a complete representation of alpha-propensity, we shall refer to it as alpha-Propensity. Note that the G-O-R Tables are based on native proteins, not short peptides. This distinction will turn out to be important.

ii. Residue Presence and Absence. Some residues, notably glutamate, are 'helix-makers', while others, notably proline, glycine and serine, are 'helix-breakers'. Helix-makers are frequently found in alpha-helices, helix-breakers infrequently. The following statistic, Residue Presence, tests whether a residue occurs more frequently in antigenic sites than at random. (a) Assign the residue in question a value of 1 and all other residues a value of 0. (b) Average these numbers over each site to produce a site statistic and add the site statistics together in the usual way to produce an overall statistic. Presence of the residue in question increases this statistic. Changing the sign of the residue values yields Residue Absence, which reflects the absence of the residue in question.

iii. The Moment. This is defined in conjunction with a set of amino acid values. The values are numbers which are assigned to the amino acids, e.g., hydrophobicity, charge, etc.. Unusual moments reflect non-random distribution of the values along the length of a site. We shall be most interested in charge Moments. (a) Divide the protein up into overlapping blocks of length $l$. (preferably using $l = 9$). (b) Assign all the residues in a block numbers indicating their signed dis tance from the center of the block. If $l$ is odd, the center residue gets a zero, the carboxy-terminus residues are labelled 1, 2, 3, ... . If $l$ is even, there is no center residue, but by analogy with the above, the residues next to the center are labelled 1/2 and -1/2, the ones next to those 3/2 and - 3/2, and so forth. (c) Multiply the numbers by the value of the amino acid occupying the position. (d) Add the resulting products together. This is the moment of the values within the block. Maximising this block statistic produces a site statistic.

The Moment of charge is large whenever either negative side-chains (Asp/Glu) are near the $NH_2$-terminus or positive side-chains (Arg/His/Lys) near the COOH-terminus. This non-random charge distribution is the one required for favorable interaction with the alpha-helical dipole and would be expected to correlate with alpha-helices.

We examine the Moments corresponding to the following amino acid values: (a) Charge: Arg = Lys = 1, His = 0.5 (His is somewhat arbitrary), Asp = Glu = -1, all others = 0; (b) Lysine Charge: Lys = -1, all others = 0; and (c) Aspartate Charge: Asp = -1, all others = 0. Arginine, Histidine and Glutamate Charges are defined analogously.

iv. COOH-terminus Lysines. The following are 1/0 statistics, i.e., statistics which take the value 1 if the site has a certain property and 0 otherwise. The 1-Ultimate Lysine is defined as follows: if the end-residue on a site is a lysine, then the site statistic is 1. Otherwise the site statistic is 0. The 2-Ultimate Lysine is similarly defined: the site receives a 1 if there is a lysine in either of the last two positions and 0 otherwise. (None of the antigenic sites in Table V has an antepenultimate lysine, so we arbitrarily terminate the series of Ultimate Lysines at 2.) The overall statistic S corresponding to the 1-Ultimate Lysine is the sum of the site statistics and is just the number of sites having lysine at their COOH-terminus. A similar relationship holds for the other Ultimate Lysines.

The next three statistics represent beta-sheets, turns and coils.

beta-Propensity. This is exactly analogous to alpha-Propensity except that we use Table 2 of Garnier, Osguthorpe and Robson, J. Mol. Biol, 120:97, 1978. Because it is the only attempt to represent beta-propensity, we shall refer to it as beta-Propensity.

Turn Propensity. This is analogous to alpha-Propensity, except that we use Table 3 of Garnier et al.

Coil Propensity. This is also analogous to alpha-Propensity, except that we use Table 4 of Garnier et al.

Correlations. For any pair of site statistics X and Y, and for any 23 sites (whether random or antigenic), we can calculate $r = Cov(X,Y)/(J_X J_Y)$, the correlation coefficient of the 23 ($X \leq Y$) pairs. $r = 1$ for perfect correlation (e.g., $X = -Y$), $r = -1$ for perfect anti-correlation (e.g., $X = Y$), and $r = 0$ if X and Y are independent. $r$ is itself an overall statistic, and its expectation $\bar{r}$ reflects the coupling of X and Y in random sites. Denote the r for the antigenic sites fo $r_0$. $r_0$ has a statistical significance which can be estimated by Monte Carlo computer experiments. Since $r_0$ reflects the coupling of X and Y over the actual antigenic sites, a statistically significant $r_0$ may reflect an (X,Y) pair which is unusually coupled within the antigenic sites.

EXAMPLE 3. Helix-Makers and -Breakers.

Alpha-helical conformation, whether amphipathic or not, should display the characteristics mentioned above. The helix-breakers proline and glycine should be infrequent ($p = 0.098$ and $p = 0.048$). The next helix-breaker tested, serine, was not statistically significant ($p = 0.683$). Similarly, the helix-maker glutamate was not present in unusual amounts ($p = 0.627$). In accord with the end of the above discussion on statistical methods, tests for helix-making and -breaking significance ended here.

EXAMPLE 4. COOH-terminal Lysines.

Lysine, appearing near the COOH-terminus of antigenic sites far more often than its frequency in proteins warrants, is often necessary for antigenic activity. The significance of the 1-and 2-Ultimate Lysines in Table II is remarkable ($p = 0.005$ and $p = 0.010$).

EXAMPLE 5. A Sample Antigenic Data Base.

Table V lists a sample of antigenic sites used in the statistical tests of the present invention. The invention is not intended to be limited thereby. The selection criteria for this particular list are: (a) the sites were reported to immunodominant in the response to a protein; (b) the sites were known to the inventors prior to February 21, 1986; and (c) the sites are less than 21 residues long. The restrictions involve arbitrary cut-offs, but were necessary (a) to close the statistical data base and (b) to localize immunodominant sites. (Antigenic sites much longer than 21 residues probably do not localize their immunodominant site sufficiently.) The entries in Table V are, for each experiment, representative of the shortest peptide capable of near-maximal T-cell stimulation. Such peptides are usually obvious from the experimental data: deletion of critical residues generally produces a precipitous drop in antigenic activity. When the experiments did not localize the end residues of an antigenic site, the criteria given in Spouge et al, J. Immunol., 138:204-212 (1987) were applied to give a definite peptide suitable for statistical testing. In the absence of a registry of immunodominant sites, these criteria were as objective as possible.

The data base includes the amino acid sequences of 12 proteins for which helper T-cell sites have been reported. All of these sites, 23 in all, are immunodominant--they predominated in the response of T-cells from animals immunized with the native protein or a large fragment, and were localized within the molecule by testing the proliferative response to short peptides (either cleavage fragments from the native molecule or synthetic peptides that are homologous to a part of the native molecule).

EXAMPLE 6.

Applying the algorithm is a major step in predicting the most probable immunodominant sites that show amphipathic helical potency. Table VI summarizes the sites predicted by the algorithm over all the sequences tested. The number and length of sites along a specific protein depend on the hydrophobicity profile of that protein. There are proteins that show a high degree of amphipathic helical potency (and contain many predicted sites), while others are poor in amphipathic segments. After having predicted all the possible amphipathic helical segments, the segments must be graded. The use of three factors is preferred for grading purposes: a) amphipathic score (particularly useful when comparing segments of the same length); b) the rarity of proline in helices in general (except near the $NR_2$-terminus), and in most of the helical antigenic sites in particular; c) the appearance of lysine at the carboxyl end in a large number of helical antigenic sites; and d) intensity of the least squares fit. We have found that lysine as the ultimate or penultimate C-terminal residue occurs much more frequently in immunodominant sites. In short, a preferred sequence contains amphipathic segments with proline, if present, only near the N-terminus, and lysine near the C-terminus.

Another possible indicator is the presence of N-glycosylation sites--these sites are indicative of a less favorable candidate for an immunodominant site, because the T-cell epitope may be masked by the carbohydrate.

EXAMPLE 7. Statistics Representing the Properties.

Block Averaging and Maximization: The site statistics chosen to represent the properties are to some extent arbitrary. To facilitate programming, many of these statistics are generated from more elementary block statistics, numbers that are attached to peptides of a fixed length (blocks) within the protein. The block statistics must then be converted into site statistics. There are at least two reasonable procedures for doing this: (a) Block Averaging and (b) Block Maximization. "Block Averaging" means averaging the block statistic over all the blocks completely contained within the antigenic site (similarly "Block Maximization"). If an antigenic site contains many 'ordinary' blocks along with some immunodominant blocks, averaging dilutes the contribution that the immunodominant blocks make to the site statistic. Hence Block Maximization is usually the procedure of choice.

This invention uses the Fauchere-Pliska scale as a measure of amino acid hydrophobicity (Fauchers et al, Eur. J. Med. Chem., 18:369 (1983)).

Note that the protein sequence is never scrambled in any way. This common (and commonly fallacious) practice is inappropriate here, since scrambled proteins do not represent possible experimental outcomes.

The emphasis is therefore more on preventing a statistical bias than on imitating a physical process Cleavage Restriction does not imitate proteolytic cleavage perfectly. For example, a tryptic digest is unlikely to produce the hypothetical site Ala-Leu-Val-Gly-Lys-Lys-Thr-Tyr-Cys-Lys because of the presence of the two internal lysines. Likewise a tryptic fragment follows a lysine or arginine in the original protein sequence. Similar considerations hold for cyanogen bromide.

In practice, the information required to eliminate experimental biases is not always available. In the absence of the requisite information, a site was always assumed to be subject to bias. The best example of this is the antigenic site Influenza Hemagglutinin 129-140 in Table V. This site was localized by examining the antigenicity of Hemagglutinin variants and a "cleavage peptide" (the cleavage method and the precise peptide were unspecified in the reference). The most conservative course is to assume that the cleavage localizing the antigenic site was tryptic, and then to subject the site to Cleavage Restriction.

Residue Restriction. Unless otherwise stated, Cleavage Restriction is always used to control the COOH-terminus of the random sites. The one exception, used in special cases only, is Residue Restriction. Here the antigenic sites are classified by their COOH-terminal residue: Arg, Lys, Met, and other. Random sites are chosen only from the same class as the corresponding antigenic site. COOH-terminal lysines will turn out to be significant correlates of antigenicity: the intent of Residue Restriction is to remove the effects of COOH-termination in lysine and measure independent effects from other sources. By including restrictions on arginine and methionine, Residue Restriction continues to prevent bias from cleavage methods.

In general, if a statistic retains its significance under Residue Restriction, its significance cannot be due to the unusual frequency of COOH-terminal lysines in Table V.

EXAMPLE 8.

Sperm whale myoglobin is analyzed for the presence of potential immunodominant sites. Since this protein is rich in amphipathic segments, only those segments which exhibit amphipathic scores above 8 are considered. The analysis is performed as follows: the amino acid sequence is converted into a sequence of hydrophobicity values according to the Fauchere-Pliska scale. For each block of length 11, the intensity of the least squares fit and the amphipathic index are calcu lated. Table VII summarizes the candidates for immunodominant sites--segments containing blocks of the desired dominant frequency (80° - 135°). The program also looks for possible N-glycosylation sites. No such sites are found in the predicted segments of sperm whale myoglobulin.

The next step is to look through the predicted segments for subsegments that show the presence of lysine of the absence of proline. The first predicted segment, residues 6-23, contains lysine at its tenth residue. Since terminating the peptide at this position will yield a peptide only 10 residues long, the only reasonable site to consider is the whole predicted segment. The second predicted region is long (residues 23-53), and contains a proline at residue 37. Since proline, when participating in alpha-helices, tends to appear at the N-terminus of the helix, it is reasonable not to start the peptide before the proline. Residue 50 is a lysine which was shown to correlate with the C-terminus of antigenic sites, and is therefore a good subsegment for synthesis (residues 36 or 37 to 50). The subsegment 23-34, ending with lysine at position 34 and not including a proline, is also a good candidate. A similar approach is taken for each of the predicted segments, directing the positioning of a peptide within a segment by locating lysine at the C-terminus whenever possible. The algorithm's success is confirmed by three of the predicted segments in myoglobin overlapping with known antigenic sites.

The algorithm also shows that segment 113-128 is a poor candidate for an immunodominant site because it contains a Pro-Gly sequence in the middle, which strongly favors a turn interrupting the helix.

## EXAMPLE 9.

A preferred computer program for co-ordinating all the elements of the present invention is shown in Figure 1.

The computer program is written in FORTRAN 77 and runs on a VAX11/780 under VMS operating system.

The program expects an input file of the following format: First two lines: start the first line with a semi-colon (;), and then add any information on the sequence (title, number of residues, etc.). These two lines are used for the user's documentation. The third and following lines include the sequence data in one letter code (starting from the second column, 36 characters in a row). Termination of the sequence is indicated by a '1'. The program accepts sequences of length up to 1000 amino acids. For longer sequences, the dimension (parameter "lenp" in the program) and the printing formats have to be changed.

Examples of the input sequence data in required format and a portion of the output filem are presented at the end of Figure 1.

The user has an option to use block length of 11 (the program then uses a threshold of 4 for this amphipathic score), or block length of 7 (for which the program uses a threshold of 8 for the amphipathic score).

TABLE I:  STATISTICAL SIGNIFICANCES, CLEAVAGE RESTRICTION

| STATISTIC | SIGNIFICANCE | |
|---|---|---|
| | P | (1-p) |
| A.  alpha-Amphipathicity | 0.017 | |
| B.  beta-Amphipathicity | 0.855 | |
| C.  alpha-Helical Properties | | |
|  i. alpha-Propensity | 0.031 | |
|  ii. Residues (Helix-Makers and -Breakers) | | |
|   a. Glutamate Presence | 0.627 | |
|   b. Proline Absence | 0.098 | |
|   c. Glycine Absence | 0.048 | |
|   d. Serine Absence | 0.683 | |
|  iii. Moment (Helical Dipole) | | |
|   a. Charge | 0.095 | |
|   b. Lysine Charge | 0.042 | |
|   c. Histidine Charge | 0.096 | |
|   d. Arginine Charge | 0.713 | |
|   e. Aspartate Charge | 0.165 | |
|   f. Glutamate Charge | 0.734 | |
|  iv. COOH-terminus Lysines | | |
|   a. 1-Ultimate Lysine | 0.005 | |
|   b. 2-Ultimate Lysine | 0.010 | |
| D.  beta-Propensity | 0.152 | |
| E.  Turn Propensity | 0.656 | |
| F.  Coil Propensity | 0.976 | (0.024) |
| G.  Segmental amphipathicity | | |
|  i. Differential Hydrophobicity | 0.843 | |
|  ii. Maximum Differential Hydrophobicity | 0.887 | |

14

## TABLE II

### COMPARISON BETWEEN A LEAST SQUARES FIT PROCEDURE AND A DISCRETE FOURIER TRANSFORM (FAUCHERE-PLISKA SCALE)

| power spectrum procedure | criterion for a stable amphipathic segment | block length | no. of predicted amphi- pathic segments | no. of predicted antigenic sites | proba- bility |
|---|---|---|---|---|---|
| least squares fit | AS $>$ 8 | 7 | 94 | 17 | 0.0025 |
| | AS $>$ 4 | 11 | 117 | 18 | 0.0006 |
| discrete Fourier transform | AS $>$ 6 | 7 | 103 | 13 | 0.1040 |
| | AS $>$ 3 | 11 | 124 | 17 | 0.0014 |

The performances of the two power spectrum procedures for the two block lengths tested (7 and 11) are compared using the Fauchere-Pliska hydrophobicity scale. Note that there is a difference of 33% between the thresholds for amphipathic scores that were required for the different procedures (at a specific block length). For each algorithm in the table, the total number of amphipathic segments over all the proteins in the data base, the total number of predicted sites out of the 23 known antigenic T-cell sites, and the probability of getting this number of matches or greater by chance alone are summarized. AS = Amphipathic score of a segment.

## TABLE III

### COMPARISON AMONG VARIOUS HYDROPHOBICITY SCALES

### (LEASE SQUARES FIT)

| criterion for a stable amphipathic segment | block length | hydro- phobicity scale | no. of predicted amphi- pathic segments | no. of predicted antigenic sites | proba- bility |
|---|---|---|---|---|---|
| AS>8 | 7 | Fauchere-Pliska | 94 | 17 | 0.0025 |
| | | Miyazawa-Jernigan | 103 | 14 | 0.1299 |
| | | Kyte-Doolitle | 92 | 13 | 0.1028 |
| | | Rose | 97 | 14 | 0.0926 |
| AS>4 | 11 | Fauchere-Pliska | 117 | 18 | 0.0006 |
| | | Miyazawa-Jernigan | 121 | 13 | 0.1234 |
| | | Kyte-Doolitle | 110 | 15 | 0.0088 |
| | | Rose | 117 | 14 | 0.0449 |

For each hydrophobicity scale listed, the total number of amphipathic segments over all the proteins in the data base, the total number of predicted sites out of the 23 known antigenic T-cell sites, and the probability of getting this number of matches or greater by chance alone are summarized. The first column indicates the criterion used for distinguishing stable amphipathic segments. AS = Amphipathic score of a segment.

TABLE IV : COMPARISON AMONG VARIOUS
PREDICTIVE ALGORITHMS USING THE FAUCHERE-PLISKA
HYDROPHOBICITY SCALE AND A LEAST SQUARES FIT

| criterion for a stable amphipathic segment | block length | number of predicted amphipathic segments | number of predicted antigenic sites | probability |
|---|---|---|---|---|
| at least 4 amphipathic blocks | 7 | 110 | 17 | 0.0094 |
| in a segment | 11 | 92 | 16 | 0.0015 |
| at least 5 amphipathic blocks | 7 | 83 | 15 | 0.0118 |
| in a segment | 11 | 77 | 15 | 0.0022 |
| AS $>$ 4 | 7 | 158 | 18 | 0.0853 |
| | 11 | 117 | 18 | 0.0006 |
| AS $>$ 8 | 7 | 94 | 17 | 0.0025 |
| | 11 | 80 | 15 | 0.0018 |

Different algorithms when applying a least squares fit as the power spectrum procedure, and using the hydrophobicity values of the Fauchere-Pliska scale are compared. For each algorithm in the table, the total number of amphipatic segments over all the proteins in the data base, the total number of predicted sites out of the 23 known antigenic T-cell sites, and the probability of getting this number of matches or greater by chance alone are summarized. AS = Amphipathic score of a segment.

TABLE V : SUMMARY OF PREDICTED KNOWN ANTIGENIC
SITES BY THE TWO MOST EFFICIENT ALGORITHMS

= 11 , AS 4          = 7 , AS 8

| PROTEIN | T-CELL ANTI-GENIC SITES | AMPHI-PATHIC SEGMENTS | AMPHI-PATHIC SCORE | AMPHI-PATHIC SEGMENTS | AMPHI-PATHIC SCORE |
|---|---|---|---|---|---|
| Sperm Whale | [1]69-78 | 64-78 | 14.2 | 64-78 | 20.6 |
| Myo-globin | 102-113 | 99-117 | 20.1 | 100-111 | 13.5 |
| | 132-145 | 128-145 | 15.3 | 126-141 | 18.0 |
| Pigeon Cyto-chrome c | 93-104 | 92-103 | 4.3 | 92-103 | 10.9 |
| Influ-enza Hemagglu-tinin | [1]109-119 | 97-120 | 35.3 | 97-117 | 33.4 |
| A/PR/8/34 ML.S. | [2]130-140 | - | - | 126-137 | 11.2 |
| | 302-313 | 291-314 | 35.1 | 295-311 | 25.5 |
| Pork Insulin | (B)5-16 | 4-16 | 5.5 | 7-16 | 9.6 |
| | [2](A)4-14 | 1-21 | 34.0 | 1-16 | 28.0 |
| Chicken Lysozyme | 46-61 | - | - | - | - |
| | 74-86 | 72-86 | 8.9 | - | - |
| | 81-96 | 86-102 | 13.1 | - | - |
| | [2]109-119 | - | - | - | - |

[1] $NH_2$-extensions of the antigenic site required for the analysis, these residues are not part of the minimal stimulating peptide. Peptide containing them retain antigenicity, however, according to the primary source.

[2] Only critical residues were reported by the primary source. The antigenic sites were considered as blocks of length 11 centered around these residues.

| PROTEIN | T-CELL ANTI-GENIC SITES | AMPHI-PATHIC SEGMENTS | AMPHI-PATHIC SCORE | AMPHI-PATHIC SEGMENTS | AMPHI-PATHIC SCORE |
|---|---|---|---|---|---|
| Chicken Oval-bumin | 323-339 | 329-346 | 18.0 | 322-332 | 9.0 |
| Hepa-titis B Virus Pre S | 120-132 | 121-135 | 8.7 | 124-136 | 15.3 |
| Foot & Mouth Virus WP 1 | 141-160 | 148-165 | 20.3 | 151-160 | 10.0 |
| Beef Cyto-chrome c | 11- 25 | 9- 29 | 22.7 | 10- 23 | 17.5 |
|  | 66- 80 | 58- 78 | 23.6 | 61- 76 | 21.8 |
| Hepa-titis B Virus Major Surface Antigen | 38-52 | 36- 49 | 7.3 | 26- 45 | 27.5 |
|  | 95-109 | - | - | - | - |
|  | 140-154 | - | - | - | - |
| λ Repres-sor Pro-tein C1 | 12- 26 | 8- 25 | 19.5 | 8 -25 | 24.1 |
| Rabies Virus-Spike Glyco-protein Pre-cursor | 32- 44 | 29- 46 | 20.2 | 32-46 | 20.4 |
|  |  |  | $p < 0.001$ |  | $p < 0.003$ |

Summarized are the 23 immunodominant antigenic sites included in the data base, indicated by their residues numbers (column 2). These numbers are the ordinal numbers of residues in the protein sequence without counting residues of the signal peptide if it appears. The table shown, for the two final algorithms (indicated at the top of columns 3-6), which antigenic sites overlap with amphipathic segments and the calculated amphipathic scores. The predicted amphipathic segments are indicated by their residue numbers. The p values at the bottom of the table refer to the probability of getting this number of matches between antigenic sites and amphipathic segments by chance alone.

## TABLE VI : AMPHIPATHIC SEGMENTS PREDICTED BY THE ALGORITHM

| PROTEIN | PREDICTED AMPHIPHATIC SEGMENTS (CENTERS OF 11-RESIDUE BLOCKS) | | RANGE OF ANGLES | AMPHIPATHIC SCORE |
|---|---|---|---|---|
| | 11- 18 | ( K) | 95.-125. | 13.9 |
| | 28- 48 | ( K) | 85.-135. | 59.5 |
| Sperm Whale | 56- 61 | ( K) | 90.-120. | 9.3 |
| Myoglobin | 69- 73 | ( K) | 100.-120. | 14.2 |
| | 89- 91 | ( PK) | 125.-125. | 5.1 |
| | 104-112 | | 80.-115. | 20.1 |
| | 118-123 | ( P ) | 80.-100. | 9.8 |
| | 129-131 | | 95.-110. | 7.2 |
| | 133-140 | ( K) | 85.-110. | 15.3 |
| | 14- 24 | ( K) | 85.-125. | 23.5 |
| | 31- 33 | ( P ) | 105.-110. | 4.8 |
| Pigeon | 43- 48 | ( K) | 125.-135. | 9.9 |
| Cytochrome c | 63- 73 | ( PK) | 80.-110. | 23.8 |
| | 97- 98 | | 110.-115. | 4.3 |
| | 12- 20 | (* K) | 85.-120. | 19.0 |
| | 28- 30 | (* ) | 105.-125. | 5.6 |
| | 32- 36 | ( K) | 80.-115. | 7.1 |
| | 42- 51 | ( PK) | 125.-135. | 23.7 |
| | 75- 80 | ( P ) | 100.-125. | 12.9 |
| Influenza | 95-100 | | 115.-135. | 12.4 |

| | | | | |
|---|---|---|---|---|
| Hemagglutinin A/PR/8/34/Mt | 102-115 | ( PK) | 100.-130. | 35.3 |
| | 131-134 | | 80.-110. | 5.3 |
| | 204-207 | ( P ) | 80.-85. | 5.7 |
| | 209-212 | ( P ) | 85.-90. | 7.1 |
| | 260-262 | | 80.-85. | 5.7 |
| | 281-287 | (*P ) | 90.-110. | 14.2 |
| | 296-309 | ( K ) | 110.-135. | 35.1 |
| | 312-320 | ( P ) | 110.-130. | 23.9 |

| | | | | |
|---|---|---|---|---|
| | 9-11 | | 80.-80. | 5.5 |
| Pork Proinsulin | 47-51 | | 110.-125. | 12.3 |
| | 68-79 | | 85.-125. | 34.0 |

| | | | | |
|---|---|---|---|---|
| | 20-25 | | 130.-135. | 12.5 |
| | 45-49 | | 85.-105. | 7.8 |
| | 77-81 | ( P ) | 85.-90. | 8.9 |
| Chicken Lysozyme | 91-97 | ( K ) | 85.-115. | 13.1 |
| | 107-108 | | 115.-120. | 4.2 |
| | 110-112 | ( K) | 105.-105. | 4.2 |
| | 118-124 | | 100.-135. | 12.9 |

| | | | | |
|---|---|---|---|---|
| | 12-20 | ( K ) | 95.-135. | 21.9 |
| | 34-40 | | 115.-135. | 14.3 |
| | 50-72 | ( K ) | 85.-130. | 58.1 |
| | 80-98 | ( K ) | 95.-135. | 52.1 |
| | 117-128 | ( PK) | 85.-110. | 30.9 |
| | 138-159 | ( P ) | 80.-110. | 53.7 |
| Chicken Ovalbumin | 184-186 | ( K ) | 80.-90. | 8.2 |
| | 212-214 | | 120.-125. | 6.0 |
| | 218-221 | ( K ) | 110.-135. | 8.6 |
| | 250-269 | ( K ) | 110.-130. | 57.1 |
| | 300-324 | (* K) | 80.-135. | 53.8 |
| | 334-341 | | 85.-120. | 18.0 |

| | | | | |
|---|---|---|---|---|
| | 55-57 | ( P ) | 115.-120. | 5.6 |
| | 83-90 | ( P ) | 85.-105. | 17.5 |
| | 110-113 | ( P ) | 85.-95. | 6.6 |

21

| | | | | |
|---|---|---|---|---|
| Hepatitis B | 126-130 | (*P ) | 80.-105. | 8.7 |
| Virus Pre S | 152-154 | ( P ) | 80.-100. | 4.3 |
| | 159-169 | ( P ) | 85.-115. | 29.6 |
| | 6- 18 | | 90.-115. | 27.8 |
| | 37- 44 | ( P ) | 80.-130. | 15.9 |
| | 46- 55 | ( P ) | 115.-130. | 26.9 |
| | 57-60 | ( P ) | 80.-135. | 5.9 |
| | 62- 67 | | 80.-115. | 12.1 |
| Foot and Mouth | 93-106 | (*PK) | 80.-120. | 35.3 |
| Virus VP 1 | 109-111 | ( P ) | 80.- 85. | 4.7 |
| | 121-130 | | 85.-100. | 22.8 |
| | 153-160 | ( P ) | 85.-100. | 20.3 |
| | 169-178 | ( K) | 95.-135. | 21.6 |
| | 189-192 | ( P ) | 85.-105. | 7.1 |
| | 14- 24 | ( K) | 85.-125. | 22.7 |
| Beef | 31- 33 | ( P ) | 105.-110. | 4.8 |
| Cytochrome c | 63- 73 | ( PK) | 80.-110. | 23.6 |
| | 97- 98 | | 105.-105. | 4.4 |
| | 6- 9 | (*P ) | 80.- 90. | 10.1 |
| | 23- 39 | | 95.-120. | 38.0 |
| | 41- 44 | | 110.-130. | 7.3 |
| Hepatitis B virus Major Surface Antigen | 65- 67 | ( P ) | 95.-100. | 6.9 |
| | 117-120 | ( P ) | 90.-135. | 7.2 |
| | 122-126 | | 85.-100. | 11.5 |
| | 182-184 | ( P ) | 100.-110. | 5.7 |
| | 205-209 | ( P ) | 95.-120. | 13.7 |
| | 13- 20 | ( K) | 95.-120. | 19.5 |
| | 48- 61 | | 85.-135. | 39.6 |
| | 63- 71 | ( K) | 80.- 95. | 16.5 |
| | 78- 92 | ( P ) | 85.-135. | 40.7 |
| λ Repressor | 107-111 | | 85.-135. | 7.9 |
| Protein CI | | | | |

|  |  |  |  |  |
|---|---|---|---|---|
|  | 119-134 | ( K) | 85.-130. | 27.6 |
|  | 173-176 | ( P) | 115.-125. | 7.5 |
|  | 196-199 |  | 90.-100. | 10.2 |
|  | 220-230 | (*PK) | 80.-105. | 24.7 |
|  | 6- 18 | ( P ) | 85.-135. | 32.1 |
|  | 22- 24 | ( P ) | 115.-130. | 5.3 |
|  | 34- 41 | (* ) | 110.-130 | 20.2 |
|  | 69- 79 | ( K) | 95.-115. | 26.7 |
|  | 89- 91 | ( P ) | 95.-105. | 6.6 |
|  | 93- 95 | ( P ) | 95.-110. | 5.9 |
| Rabies Virus | 111-115 | ( P ) | 85.-105. | 11.9 |
| Spike | 119-128 | ( K) | 95.-130. | 21.5 |
| Glycoprotein | 142-144 | ( P ) | 110.-125. | 6.0 |
| Precursor | 146-148 | ( P ) | 80.-105. | 5.2 |
|  | 198-203 |  | 105.-120. | 14.0 |
|  | 215-220 | ( K) | 85.-105. | 15.2 |
|  | 228-232 |  | 135.-135. | 9.1 |
|  | 255-262 |  | 110.-135. | 17.7 |
|  | 270-273 |  | 80.- 85. | 5.5 |
|  | 284-292 | ( K) | 95.-120. | 21.5 |
|  | 297-315 | ( K) | 95.-130. | 48.0 |
|  | 330-338 | ( PK) | 80.-120. | 23.7 |
|  | 345-348 | ( P ) | 95.-110. | 8.5 |
|  | 377-381 |  | 80.- 95. | 9.0 |
|  | 383-388 |  | 100.-135. | 12.1 |
|  | 398-403 | ( PK) | 100.-115. | 11.3 |
|  | 409-414 |  | 110.-115. | 10.3 |
|  | 416-418 | ( P ) | 80.-135. | 4.0 |
|  | 422-426 |  | 105.-125. | 11.2 |
|  | 436-441 |  | 125.-135. | 11.4 |
|  | 461-467 | (*P ) | 85.-130. | 13.6 |
|  | 489-494 | ( K) | 100.-115. | 12.5 |
|  | 496-498 |  | 115.-135. | 5.5 |

Table VI summarizes the predicted amphipathic segments along the 12 sequences in the data base. Predictions were made by running the algorithm and setting the threshold to 4. The second column

23

indicates the predicted segments by the midpoint positions of the predicted 11-residue blocks. Hence. the predicted sites extend 5 residues to each slide, as, for example, in the amphipathic segments listed in Table V. The third column indicates the range of dominant frequencies for the blocks contained in the segment. When proline is present within the last 10 residues at the C-terminus of the predicted segment it is indicated by a P to the right of the second column. Presence of lysine after the first 10 residues at the N-terminus of the predicted segment is indicated by a K to the right of the second column. Potential N-glycosylation sites are indicated to the right of the second column by an asterisk. In pork proinsullin, residues 1 to 29 correspond to the B chain and residues 64 to 94 correspond to the A chain of insulin.

## TABLE VII : PREDICTED AMPHIPATHIC SEGMENTS IN SPERM WHALE MYOGLOBIN

| MID POINTS OF BLOCKS | SEQUENCE | RANGE OF ANGLES | AMPHIPATHIC SCORE |
|---|---|---|---|
| 11- 18 | WQLVLHVWAKVEADVA GH | 95 - 125 | 13.9 |
| 28- 48 | GHGQDILIRLFKSHPE TLEKFDRFKHLKTEA | 85 - 135 | 59.5 |
| 56- 61 | TEAEMKASEDLKKHGV | 90 - 120 | 9.3 |
| 69- 73 | HGVTVLTALGAILKK | 100 - 120 | 14.2 |
| 104-112 | IPIKYLEFISEAIIH VLHS | 80 - 115 | 20.1 |
| 118-123 | HVLHSRHPGDFGADAQ | 80 - 100 | 9.8 |
| 133-140 | QGAMNKALELFRKDIA AK | 80 - 110 | 15.3 |

Table VII summarizes the sites predicted by the algorithm for sperm whale myoglobin, when setting the threshold of the amphipathic score to be 8. The first column indicates the midpoints of the predicted blocks. The second column lists the appropriate sequences in the usual one letter code notation. Possible C-terminal lysines and presence of proline are marked. The third column indicates the range of dominant frequencies for the blocks contained in the segment.

## TABLE VIII: CORRELATION AND STATISTICAL SIGNIFICANCES, CLEAVAGE RESTRICTION

| STATISTICS X AND Y | | EXPECTED CORRELATION $\bar{r}$ | ANTIGENIC CORRELATION $r_0$ | SIGNIFICANCE p |
|---|---|---|---|---|
| X. | alpha-Amphipathicity | | | |
| Y. | alpha-Propensity | −0.260 | −0.479 | 0.136 |
| X. | alpha-Propensity | | | |
| Y. | beta-Propensity | −0.368 | −0.652 | 0.954 |
| X. | beta-Propensity | | | |
| Y. | Turn Propensity | 0.082 | 0.452 | 0.041 |

**Claims**

1. A method for predicting segments of protein sequences which are likely to be recognized by T lymphocytes comprising assigning hydrophobicity values to each amino acid in a protein sequence and determining which regions of amino acids are able to form an amphipathic helix.

2. A method of predicting antigenic sites of a protein which are recognized by T-cells, comprising:

a. determining the amphipathicity of segments along the entire sequence of said protein;

b. determining the conformational propensity of segments along the entire sequence of said protein;

c. determining the presence or absence of helix-breakers in segments along the entire sequence of said protein;

d. determining the presence and location in the protein sequence of amino acid residues which favor T-cell recognition; and

predicting a propensity of a protein sequence to be recognized by T-cells based on the properties described in a, b, c, and d.

3. The computer program as shown in Figure 1.

4. A computer programmed substantially in accordance with the coding sequence listed in Figure 1.

```
                         FIG. 1
C                     PROGRAM AMPHI
C                     * * * * * * * * * * * *
C        * * * * * * * * * * * * * * * * * * * * * * * * * * * * * * * * * * * * * * * * * * * *
C        PREDICTION OF HELPER T-CELL  ANTIGENIC SITES THAT
C        CORRELATE WITH AMPHIPATHIC HELICES
C        * * * * * * * * * * * * * * * * * * * * * * * * * * * * * * * * * * * * * * * * * * * *
C
C
         PARAMETER LENP=1000
         DIMENSION VAL(LENP),HYDRO1(21),VAR(LENP),LET(80).
       1    ANG(LENP),IANG(LENP),IS1(LENP),AMPH1(LENP).
       1    AMPH2(LENP),HYDRO2(21),MANG(LENP)
         INTEGER SEQ(LENP),SEQ1(LENP).DEL
       1    .AMINO(21),AMINO1(21)
         CHARACTER*30 DEF3.FILE1,FILE2
         CHARACTER*1 IFLAG1.IFLAG2.IFLAG3
         DATA AMINO/'A','R','N','D','C','Q','E','G',
       1      'H','T','L','K','M','F','P','S','T','W','Y','V','X'/
         DATA AMINO1/'ALA','ARG','ASN','ASP','CYS','GLN','GLU',
       1      'GLV','HIS','ILE','LEU','LYS','MET','PHE','PRO'
       1    ,'SER','THR','TRP','TYR','VAL',XXX'/
         DATA HYDRO1/0,31,-1.01,-O.60.-0.77,1.54,-0.22,-0.64,0.00,
       1 0.13,1.80,1.70.-0.99,1.23,1.79,0.72.-0.04,0.26,2.25,0.96,
       1 1.22,0.00/
         DATA HYDRO2/0.,-1.,-1.,-1.,1.,
       1  -1.,-1.,0.,0.,1.,1.,-1.,1.,1.,1.,0.,0.,1.,1.,1.,0./
C        ————————————————————————
         PRINT 111
 111     FORMAT(1X,'ENTER INPUT FILE NAME')
         READ 112,FILE1
         PRINT 113
 113     FORMAT(1X,'ENTER OUTPUT FILE NAME')
         READ 112,FILE2
 112     FORMAT(A)
         OPEN(UNIT=5,FILE=FILE1 ,STATUS='OLD')
         OPEN(UNIT=6,FILE=FILE2 ,STATUS='NEW')
         PRINT 117
 117     FORMAT(1X,'CHOOSE BLOCK LENGTH: 7 OR 11')
         READ *, LSEG
         IF (LSEG.EQ.11)TH=4.
         IF(LSEG.EQ.7)TH=8.
C
C        INITIALIZATION OF VARIABLES
C
         DO 100 I=1,LENP
         VAL(I)=0.
         SEQ1(I)='XXX'
         SEQ(I)=0
         VAR(I)=0.
         MANG(I)=0
         AMPH1(I)=0.
         AMPH2(I)=0.
         IS1(I)=0
 100     CONTINUE
```

# 0 279 994

FIG.1 (CONT.)

```
C
C    READING THE SEQUENCE DATA AND CONVERTING THE SEQUENCE
C    INTO A SEQUENCE OF HYDROPHOBICITY VALUES
C
     LSEQ=0
     DEF1=' '
     DO WHILE(DEF1.NE.';')
     READ(5,10,END=1000)DEF1,DEF3
     END DO
10   FORMAT(A1,A30)
     READ (5,20)CHAR
20   FORMAT(A)
     LCHECK=0
     DO WHILE (LCHECK.NE.1)
     READ(5,30,END=1000)(LET(J),J=1,72)
     DO 200 J=1,72
     IF(LET(J).EQ.'1')LCHECK=1
     IF(LET(J).EQ.' ')GO TO 200
     LSEQ=LSEQ+1
     IF(LSEQ.GT.0)THEN
     DO 300 K=1,21
     IF(LET(J).EQ.AMINO(K)) THEN
     SEQ(LSEQ)=K
     SEQ1(LSEQ)=AMINO1(K)
     VAL(LSEQ)=HYDRO1(K)
     ELSE
     ENDIF
300  CONTINUE
     ELSE
     ENDIF
200  CONTINUE
     END DO
     LEN=LSEQ-1
30   FORMAT(72A1)
50   FORMAT(/A40,2X,I3//)
     DEL=LSEG/2
     WRITE(6,50)DEF3,LEN
C
C    LEAST SQUARES FIT
C
     CALL PERIOD(VAL,LSEG,LEN.VAR.IANG.AMPH1.AMPH2)
C
C    ASSIGNING A '1' TO BLOCKS THAT SHOW A HELICAL
     AMPHIPATHICITY
C
     NBLOCK=LEN-LSEG+1
     DO 710 II=1,NBLOCK
     IF(IANG(II).GE.17.AND.IANG(II).LE.28)MANG(II)=1
     ANG(II)=(IANG(II)-1)*5
710  CONTINUE
C
C    ASSIGNING '0' TO AMPHIPATHIC BLOCKS THAT INCLUDE MORE
C    THAN 5 CONTIGUOUS RESIDUES ,ALL HYDROPHOBIC OR ALL
     HYDROPHILIC.
C
     DO 711 II=1,NBLOCK
```

FIG.1 (CONT.)

```
      NSAME=0
      DO 712 IJ=II,II+LSEG-2
      IF ((HYDRO2(SEQ(IJ)).EQ.1..AND.HYDRO2(SEQ(IJ+1)).EQ.1.).OR.
     1 (HYDRO2(SEQ(IJ)).EQ.-1..AND.HYDRO2(SEQ(IJ+1)).EQ.-1.))THEN
      NSAME=NSAME+1
      ELSE
      NSAME=0
      ENDIF
      IF(NSAME.EQ.4)THEN
      MANG(II)=0
      GO TO 711
      ELSE
      ENDIF
712   CONTINUE
711   CONTINUE
C
C     OPTION TO PRINT A DETAILED OUTPUT
C
      PRINT 114
114   FORMAT(1X,'IF YOU WOULD LIKE A DETAILED OUTPUT-TYPE 1
     1 ELSE-TYPE O')
      READ 115, IOUT
115   FORMAT(I1)
      IF(IOUT.EQ.1)THEN
      WRITE(6,51)
51    FORMAT(2X,'BLOCK',4X,' MID POINT OF BLOCKS ',2X, 'THETA'
     1 ,2X,'I(THETA)',4X,' A1 ',3X' A2 ', /13X,'——————————'
     1 /8X,'RES NO. RES.      HYD.')

      WRITE96,902)(II,SEQ1(II),VAL(II),II=1,DEL)
      DO 116 II=1,NBLOCK
      WRITE(6,901)II,II+LSEG-1,II+DEL.SEQ1(II+DEL)
     1 ,VAL(II+DEL),ANG(II),VAR(II),MANG(II),AMPH1(II),AMPH2(II)
116   CONTINUE
      WRITE(6,902)(II,SEQ1(II),VAL(II),II=NBLOCK+DEL+1,LEN)
      ELSE
      ENDIF
C
C     DETECTING AMPHIPATHIC SEGMENTS WITH AMPIIIPATHIC
      SCORE>THRESHOLD
C
      WRITE(6,7919)
7919  FORMAT(//8X,'PREDICTED AMPHIPATHIC SEGMENTS'//
     1 6X,'MID POINTS',2X,' ANGLES',5X,'  AS '/6X,'OF BLOCKS'/
     1       ——————————————————————————'/)
      NUM=0
      S1=0.
      DO 6000 K=1,NBLOCK+1
      IF (MANG(K).EQ.1)THEN
      NUM=NUM+1
      S1=S1+MAX(AMPH1(K),AMPH2(K))
      ELSE
          IF(S1.GE.TH)THEN
          INI=K-NUM
          ANG1=0.
          ANG2=180.
```

FIG. 1 (CONT.)

```
        SCORE1=0.
        SCORE2=0.
        DO 6001 II=INI,INI+NUM-I
        ISI(II)=1
        ANG1=MAX(ANG1,ANG(II))
        ANG2=MIN(ANG2,ANG(II))
        SCORE=MAX(AMPH1(II),AMPH2(II))
        SCORE2=SCORE2+SCORE
        SCORE1=MAX(SCORE,SCORE1)
6001    CONTINUE
C
C       CHECKING POSSIBLE N GLYCOSYLATION SITES
C       AND PRESENCE OF PROLINE OR LYSINE
C
        IFLAG1=' '
        IFLAG2=' '
        IFLAG3=' '
        DO 6010 II-INI,INI+NUM-3+LSEG-1
        IF(SEQ1(II).EQ.'ASN'.AND. SEQ1(II+1).NE.'PRO'
     1          AND.(SEQ1(II+2).EQ.'SER'.OR.SEQ1(II+2).EQ.'THR'))
     1          IFLAG1='*'
6010            CONTINUE
        IF(INI+10.LE.K-1+LSEG-1)THEN
        DO 6011 II=INI+10,K-1+LSEG-1
        IF(SEQ(II).EQ.'LYS')IFLAG2='K'
6011    CONTINUE
        ELSE
        ENDIF
        DO 6012 II=K-1+LSEG-1,K-1+LSEG-1-9,-1
        IF(II.GT.0.AND.SEQ1(II).EQ.'PRO')IFLAG3='P'
6012    CONTINUE
        WRITE (6,7000)IFLAG1,IFLAG2,IFLAT3,
     1          INI+DEL,INI+NUM-1+DEL,ANG2,ANG1,SCORE2
        ELSE
        ENDIF
        NUM=0
        S1=0.
        ENDIF
6000    CONTINUE
7000    FORMAT(3(1X,A1),1X,I3,'-',I3,3X,F4,0,'-',F4,0,3X,F5,1)
C
C       STATISTICS
C
        ITOTAL=0
        DO 6002 II=1.NBLOCK
        IF(IS1(II).EQ.1)ITOTAL=ITOTAL+1
6002    CONTINUE
6004    FORMAT(/1X,'NO. OF PREDICTED BLOCKS',I5)
        WRITE(6,6004)ITOTAL
902     FORMAT(11X,I3,3X,A3,6X,F6,3)
901     FORMAT(1X,I3,'-',I3,3X,I3,3X,A3,6X,F6,3,3X,F4.0,4X,F5,2,4X,I1
     1    ,2(2X,F5,2))
700     CONTINUE
1000    END
C————————————————————————————————————————
C
```

FIG. 1 (CONT.)

```
C     ****************************************************************
C     *   SUBROUTINE FOR THE LEAST SQUARES FIT CALCULATIONS      *
C     *   RETURNS TO THE MAIN PROGRAM THE MAXIMUM INTENSITY      *
C     *   ANGLE OF MAXIMUM INTENSITY AND THE TWO AMPHIPATHIC     *
C     *   INDICES FOR ALL THE BLOCKS                             *
C     ****************************************************************
C
      SUBROUTINE PERIOD (PSEQ,LSEG,LSEQ,RMAX,IANG,AMPH1,AMPH2)
      DIMENSION H(37,1000),PSEQ(1000),RMAX(1000)
     1   .IANG(1000),AMPH1(1000),AMPII2(1000),HH(0:30)
      REAL*8 CC(37),SS(37),C1(37),CS(37,-5:5),SN(37,-5:5),P(37)
     1    ,PI,HM1,HK1,HKM1,HK2,SK2,SK4,S

      DO 100 I=1,1000
      DO 100 K=1,37
100   H(K,I)=0.0

      FL=FLOAT(LSEG)
      L2=LSEG/2
C
C        SINE/COSINE CALCULATIONS
C
      PI=4.0D00*DATAN(1.0D00)

      DO 170 J=1.37
      CC(J)=0.D00
      SS(J)=0.D00
      C1(J)=0.D00
      P(J)=P1*(J-1)/(36.0D00)
      DD 165 K=-5,5
      CS(J,K)=DCOS(P(J)*K)
      SN(J,K)=DSIN(P(J)*K)
      CC(J)=CC(J)+CS(J,K)*CS(J,K)
      SS(J)=SS(J)+SN(J,K)*SN(J,K)
      C1(J)=C1(J)+CS(J,K)
165   CONTINUE
170   CONTINUE

      SK2=110.
      SK4=1958.
      S=SK4-SK2*SK2/FL
C
C        SUBTRACTION OF HBAR
C
      DO 200 I=1,LSEQ-LSEG+1
          DO 205 K=0,LSEG-1
                HH(K)=PSEQ(I+K)
205       CONTINUE
      HBAR=0.0
      DO 20 K=0,LSEG-1
          HBAR=HBAR+HH(K)
20    CONTINUE
      HBAR=HBAR/FL
      DO 30 K=0,LSEG
          HH(K)=HH(K)-HBAR
30    CONTINUE
```

0 279 994

FIG. 1 (CONT.)

```
C
C      LEAST SQUARES FIT CALCULATIONS
C
       HM1=0.0D00
       HK1=0.0
       HKM1=0.0
       HK2=0.0
       DO 40 K=-L2,L2
           HM1=HM1+HH(K+L2)*(-1.0)**K
           HK1=HK1+HH(K+L2)*K
           HKM1=HKM1+HH(K+L2)*K*(-1.0)**K
           HK2=HK2+HH(K+L2)*K**2
40     CONTINUE

       A=HK2*HK2/S
       B=HK1*HK1/SK2
       H(1,I)=A+B
       DO 60 J=2.36
           HC=0.0
           HS=0.0
           DO 50 K=-L2,L2
                   HC=HC+HH(K+L2)*CS(J.K)
                   HS=HS*HH(K+L2)*SN(J.K)
50         CONTINUE

           A=HC*HC/(CC(J)-C1(J)*C1(J)/FL)
           B=HS*HS/SS(J)
           H(J,I)=A+B

60     CONTINUE

       A=HM1*HM1/(FL-1.0/FL)
       B=HKM1*HKM1/SK2
       H(37,I)=A+B
200    CONTINUE
C
C      SEARCHING FOR MAXIMUM INTENSITY
C
       DO 400 I-1,LSEQ-LSEG+1
       SUMH=0.
       RMAX(I)=0.
       DO 401 JJ=1,37
       RMAX(I)=MAX(RMAX(I),H(JJ,I))
       IF(RMAX(I),EQ,H(JJ,I))IANG(I)=JJ
       SUMH=SUMH+H(JJ,I)
401    CONTINUE
C
C      CALCULATING THE AMPHIPATHIC INDICES
C
       RNUM=0.
       DO 402 JJ=18,23
402    RNUM=RNUM+H(JJ,I)
       IF(SUMH.NE.O.)AMPH1(I) = RNUM/SUMH*37./6.
       RNUM=0.
       DO 403 JJ=22,28
403    RNUM=RNUM+H(JJ,I)
       IF(SUMH.NE.O.)AMPH2(I) = RNUM/SUMH*37./7.
```

## FIG. 1 (CONT.)

```
400  CONTINUE
     RETURN
     END
```

### INPUT FILE

;the sperm whale sequence is shown.

MVWHP           153 amino acids

V L S E G E W Q L V L H V W A K V E A D V A G H G Q D I L I B L F K S H

P E T L E K F D R F K H L K T E A E M K A S E D L K K H G V T V L T A L

G A I L K K K G H H E A E L K P L A Q S H A ¯ K H K I P I K V L E F I S

E A I I H V L H S R H P G D F G A D A Q G A M N K A L E L F R K D I A A

K Y K E L G V Q G1

### OUTPUT FILE

The sperm whale sequence is sh 153

| BLOCK | MID POINT OF BLOCKS | | | THETA | I(THETA) | | A1 | A2 |
|-------|---------|------|-------|-------|----------|---|------|------|
|       | RES NO. | RES. | HYD.  |       |          |   |      |      |
|       | 1       | VAL  | 1.220 |       |          |   |      |      |
|       | 2       | LEU  | 1.700 |       |          |   |      |      |
|       | 3       | SER  | -0.040 |      |          |   |      |      |
|       | 4       | GLU  | -0.640 |      |          |   |      |      |
|       | 5       | GLV  | 0.000 |       |          |   | *    |      |
| 1-11  | 6       | GLU  | -0.640 | 35.  | 4.80     | 0 | 0.29 | 0.32 |
| 2-12  | 7       | TRP  | 2.250 | 160.  | 5.40     | 0 | 0.54 | 0.38 |
| 3-13  | 8       | GLN  | -0.220 | 165. | 4.63     | 0 | 0.44 | 0.49 |
| 4-14  | 9       | LEU  | 1.700 | 165.  | 5.58     | 0 | 1.08 | 0.41 |
| 5-15  | 10      | VAL  | 1.220 | 155.  | 5.26     | 0 | 1.34 | 1.15 |
| 6-16  | 11      | LEU  | 1.700 | 105.  | 6.10     | 1 | 1.52 | 0.89 |
| 7-17  | 12      | HIS  | 0.130 | 95.   | 7.03     | 1 | 2.73 | 0.99 |
| 8-18  | 13      | VAL  | 1.220 | 100.  | 3.63     | 1 | 1.41 | 1.19 |
| 9-19  | 14      | TRP  | 2.250 | 125.  | 3.84     | 1 | 1.29 | 1.63 |
| 10-20 | 15      | ALA  | 0.310 | 100.  | 4.37     | 1 | 1.61 | 1.18 |
| 11-21 | 16      | LYS  | -0.990 | 100. | 4.34     | 1 | 1.67 | 1.24 |
| 12-22 | 17      | VAL  | 1.220 | 95.   | 3.85     | 1 | 1.73 | 0.79 |
| 13-23 | 18      | GLU  | -0.640 | 95.  | 4.06     | 1 | 1.64 | 0.66 |
| :     | :       |      |       |       |          |   |      |      |
| :     | :       |      |       |       |          |   |      |      |
| :     | :       |      |       |       |          |   |      |      |
| :     | :       |      |       |       |          |   |      |      |
| :     |         |      |       |       |          |   |      |      |
| 131-141 | 136   | GLU  | -0.640 | 100. | 6.58     | 1 | 1.81 | 1.35 |
| 132-142 | 137   | LEU  | 1.700 | 100.  | 7.57     | 1 | 1.95 | 0.84 |
| 133-143 | 138   | PHE  | 1.790 | 95.   | 6.67     | 1 | 1.92 | 1.16 |
| 134-144 | 139   | ARG  | -1.010 | 95.  | 4.63     | 1 | 1.57 | 0.71 |
| 135-145 | 140   | LYS  | -0.990 | 85.  | 6.52     | 1 | 1.18 | 0.53 |
| 136-146 | 141   | ASP  | -0.770 | 75.  | 10.41    | 0 | 1.31 | 0.31 |
| 137-147 | 142   | ILE  | 1.800 | 65.   | 6.45     | 0 | 1.17 | 0.25 |
| 138-148 | 143   | ALA  | 0.310 | 90.   | 5.74     | 1 | 1.83 | 0.46 |
| 139-149 | 144   | ALA  | 0.310 | 50.   | 4.71     | 0 | 1.55 | 0.93 |
| 140-150 | 145   | LYS  | -0.990 | 105. | 6.24     | 1 | 2.11 | 1.30 |
| 141-151 | 146   | TYR  | 0.960 | 165.  | 5.09     | 0 | 1.12 | 1.17 |
| 142-152 | 147   | LYS  | -0.990 | 155. | 4.30     | 0 | 0.94 | 0.74 |
| 143-153 | 148   | GLU  | -0.640 | 140. | 4.71     | 0 | 0.54 | 1.63 |

FIG. 1 (CONT.)

|       |      |        |
|-------|------|--------|
| 149   | LEU  | 1.700  |
| 150   | GLY  | 0.000  |
| 151   | TYR  | 0.960  |
| 152   | GLN  | -0.220 |
| 153   | GLY  | 0.000  |

SUMMARY TABLE

PREDICTED AMPHIPATHIC SEGMENTS

| | MID POINTS OF BLOCKS | ANGLES | AS |
|-----|------|------------|------|
| K   | 11- 18   | 95.-125.   | 13.9 |
| K   | 28- 48   | 85.-135.   | 59.5 |
| K   | 58- 61   | 90.-120.   | 9.3  |
| K   | 69- 73   | 100.-120.  | 14.2 |
| K P | 89- 91   | 125.-125.  | 5.1  |
|     | 104-112  | 80.-115.   | 20.1 |
| P   | 118-123  | 80.-100.   | 9.8  |
|     | 129-131  | 95.-110.   | 7.2  |
| K   | 133-140  | 85.-110.   | 15.3 |

NO. OF PREDICTED BLOCKS        67

Comments on the output

There is an option to obtain a detailed output that includes the results for each block of 11 residues and a summary table of the predicted antigenic sites, or to obtain only a summary table.

Detailed output

The seventh column indicates whether the block is predicted to be of helical periodicity. A " 1 " indicates helical periodicity.

Summary Table

Presence of lysine after the first block at the N-terminus of the predicted segment is indicated by a K to the left of the first column. Presence of proline within the first block at the C-terminus of the predicted segment is indicated by a P to the left of the first column. Possible N-glycosylation sites are indicated by an *.

FIG. 1 (CONT.)

| 149 | LEU | 1.700 |
| 150 | GLY | 0.000 |
| 151 | TYR | 0.960 |
| 152 | GLN | −0.220 |
| 153 | GLY | 0.000 |

SUMMARY TABLE

PREDICTED AMPHIPATHIC SEGMENTS

| | MID POINTS OF BLOCKS | ANGLES | AS |
|---|---|---|---|
| K | 11- 18 | 95.-125. | 13.9 |
| K | 28- 48 | 85.-135. | 59.5 |
| K | 58- 61 | 90.-120. | 9.3 |
| K | 69- 73 | 100.-120. | 14.2 |
| K P | 89- 91 | 125.-125. | 5.1 |
| | 104-112 | 80.-115. | 20.1 |
| P | 118-123 | 80.-100. | 9.8 |
| | 129-131 | 95.-110. | 7.2 |
| K | 133-140 | 85.-110. | 15.3 |

NO. OF PREDICTED BLOCKS     67

Comments on the output

There is an option to obtain a detailed output that includes the results for each block of 11 residues and a summary table of the predicted antigenic sites, or to obtain only a summary table.

Detailed output

The seventh column indicates whether the block is predicted to be of helical periodicity. A " 1 " indicates helical periodicity.

Summary Table

Presence of lysine after the first block at the N-terminus of the predicted segment is indicated by a K to the left of the first column. Presence of proline within the first block at the C-terminus of the predicted segment is indicated by a P to the left of the first column. Possible N-glycosylation sites are indicated by an *.